# EUROPEAN PATENT SPECIFICATION

(11) **EP 4 297 723 B1**
(45) Date of publication and mention of the grant of the patent: **20.05.2026**
(21) Application number: 22760580.5
(22) Date of filing: 28.02.2022
(51) Int. Cl.: A61K 9/00, A61M 5/158, A61M 37/00

(54) **MICRONEEDLE COMPOSITIONS AND METHODS OF DELIVERY USING THE SAME**
MIKRONADELZUSAMMENSETZUNGEN UND VERFAHREN ZUR FREISETZUNG DAMIT
COMPOSITIONS DE MICRO-AIGUILLES ET MÉTHODES D'ADMINISTRATION LES UTILISANT

(30) Priority: 27.02.2021 US 202163154688 P
(43) Date of publication of application: 03.01.2024
(60) Divisional application: 26172524.6
(73) Proprietor: The Brigham & Women's Hospital, Inc., Boston, Massachusetts 02115 (US); MASSACHUSETTS INSTITUTE OF TECHNOLOGY, Cambridge, MA 02142-1324 (US)
(72) Inventor: ARTZI, Natalie, Brookline, Massachusetts 02445 (US); DOSTA, Pere, Cambridge, Massachusetts 02139 (US); PUIGMAL, Nuria, Brookline, Massachusetts 02446 (US)
(74) Representative: Forresters IP LLP
(86) International application number: PCT/US2022/018192
(87) International publication number: WO 2022/183122

(56) References cited:
- EP-A2- 1 544 216
- WO-A1-2018/017674
- WO-A2-2008/011625
- CN-B- 105 596 294
- US-A1- 2018 133 447
- US-A1- 2020 377 929
- US-A1- 2022 023 605
- LAFFLEUR F ET AL: "Chemical Modification of Hyaluronic Acid for Intraoral Application", JOURNAL OF PHARMACEUTICAL SCIENCES, vol. 103, no. 8, 1 July 2014 (2014-07-01), pages 2414 - 2423, XP093232340, DOI: https://doi.org/10.1002/jps.24060
- LI ET AL.: "Redox-sensitive micelles self-assembled from amphiphilic hyaluronic acid deoxycholic acid conjugates for targeted intracellular delivery of paclitaxel", BIOMATERIALS, vol. 33, no. 7, 12 December 2011 (2011-12-12), pages 2310 - 2320, XP028442480, Retrieved from the Internet <URL:https://www.sciencedirect.com/science/article/abs/pii/S0142961211013767?via%3Dihub> [retrieved on 20220510], DOI: 10.1016/j.biomaterials.2011.11.022

## Description

### CLAIM OF PRIORITY

This application claims the benefit of U.S. Provisional Application Serial No. 63/154,688, filed on February 27, 2021.

### TECHNICAL FIELD

The present disclosure describes compositions comprising degradable hyaluronic-based hydrogels including microneedle arrays and methods of preparing the same. The disclosure also describes methods of delivering a therapeutic agent in a subject in need thereof using these compositions. The hydrogel compositions can include an amino-modified hyaluronic acid polymer comprising a disulfide bond. The methods of delivering a therapeutic agent can include contacting a skin surface of the subject with the microneedle array compositions and applying pressure such that the tips of the plurality of microneedles perforate and/or penetrate the skin surface, thereby releasing the therapeutic agent in the tissue.

### BACKGROUND

Microneedles (MN) have become an emerging tool for biomedical applications, attracting both scientific and industrial interests. Despite being a sought-after technology, MN-based platforms displaying both therapeutic and diagnostic capacities within the same device have rarely been explored as synchronizing both capacities has been proven challenging. Accordingly, there is an unmet need for MN-based platforms capable of therapeutic and diagnostic applications.

### SUMMARY

Certain aspects of the present disclosure are directed to microneedle arrays comprising: a plurality of microneedles projecting from a substrate, each microneedle of the plurality of microneedles comprising a penetrating tip and a base that is integrally connected with the substrate, wherein each microneedle of the plurality of microneedles is a porous microneedle composed of a degradable hyaluronic acid polymer comprising a disulfide bond coupled to a terminal amine group, wherein the hyaluronic acid polymer is crosslinked via the terminal amine group.

In some embodiments, each microneedle of the plurality of microneedles has a height of about 100 µm to about 1,500 µm and a base having a radius of about 100 µm to about 1,500 µm. In some embodiments, each microneedle has a height of about 600 µm and a base having a radius of about 150 µm. **In** some embodiments, each microneedle of the plurality of microneedles has a pyramidal or conical shape. In some embodiments, the substrate is a polymeric, biodegradable substrate. **In** some embodiments, the polymeric, biodegradable substrate comprises poly(D,L-lactide-co-glycolide) polymer. In some embodiments, each microneedle of the plurality of microneedles further comprises a therapeutic agent.

In some embodiments, the therapeutic agent comprises a chemokine, a chemotherapeutic, a nucleic acid, a protein, a macromolecule, a nanoparticle, a chemical-based drug, or any combination thereof.

Certain aspects of the present disclosure are directed to methods of preparing a microneedle array, the method comprising: providing a hyaluronic acid polymer solution comprising a disulfide bond coupled to a terminal amine group; casting the hyaluronic acid polymer solution into a microneedle mold; optionally centrifuging the microneedle mold containing the hyaluronic acid polymer solution; freeze-drying the microneedle mold containing the hyaluronic acid polymer solution; casting a crosslinker into the microneedle mold containing the freeze-dried hyaluronic acid polymer; centrifuging the microneedle mold containing the freeze-dried hyaluronic acid polymer and the crosslinker, thereby crosslinking the hyaluronic acid polymer and forming a hydroge, wherein the hyaluronic acid polymer is crosslinked via the terminal amine group; and optionally freeze-drying the microneedle mold containing the hyaluronic acid hydrogel.

In some embodiments, the methods further comprise depositing a solution comprising one or more therapeutic agents after optionally freeze-drying the microneedle mold containing the hyaluronic acid hydrogel. In some embodiments, the solution comprising the one or more therapeutic agents further comprises glycine. In some embodiments, the methods further comprise optionally centrifuging and/or applying vacuum to the mold containing the freeze-dried hyaluronic acid hydrogel and the solution. In some embodiments, the methods further comprise adding a backing layer to the microneedle mold containing the freeze-dried hyaluronic acid hydrogel and the solution. In some embodiments, the backing layer comprises a biodegradable polymer. In some embodiments, the biodegradable polymer comprises a poly(D,L-lactide-co-glycolide) polymer. In some embodiments, the methods further comprise drying the microneedle mold containing the hyaluronic acid hydrogel and the backing layer.

In some embodiments, the crosslinker is polyethylene glycol (PEG) comprising a succinimidyl functional group. In some embodiments, the PEG has a molecular weight ranging from about 10 kDa to about 40 kDa. In some embodiments, the chemical crosslinker comprises a first PEG having a molecular weight of about 40 kDa and a second PEG having a molecular weight of about 10 kDa. In some embodiments, the chemical crosslinker comprises the first PEG and the second PEG at a ratio of about 0:100 wt% to about 100:0 wt%. In some embodiments, the methods further comprise removing the microneedle mold, thereby providing the microneedle array.

Certain aspects of the present disclosure are directed to methods of transdermally delivering a therapeutic agent to a subject in need thereof, the method comprising: contacting the microneedle arrays of the disclosure with a skin surface of the subject; and applying pressure on the microneedle array such that the penetrating tip of each microneedle of the plurality of microneedles penetrates the skin surface, thereby releasing the therapeutic agent.

Methods of treatment are not according to the invention as claimed.

In some embodiments, the methods further comprise maintaining the microneedle array in place for about 5 minutes to about 24 hours after the penetrating tip of each microneedle of the plurality of microneedles penetrates the skin surface. In some embodiments, the methods further comprise sampling a skin interstitial fluid of the subject, wherein the sampling step comprises extracting the skin interstitial fluid with the plurality of microneedles. In some embodiments, the interstitial fluid comprises a biomarker and/or a cell. In some embodiments, the therapeutic agent comprises a cell, a chemokine, a chemotherapeutic, a nucleic acid, a protein, a macromolecule, a nanoparticle, an exosome, a chemical-based drug, or any combination thereof.

Certain aspects of the present disclosure are directed to methods of sampling an interstitial fluid of a subject in need thereof, the method comprising: contacting the microneedle array of the disclosure with a skin surface of the subject; and applying pressure on the microneedle array such that the penetrating tip of each microneedle of the plurality of microneedles penetrates the skin surface, thereby absorbing the interstitial fluid.

In some embodiments, the methods further comprise removing the microneedle array from the skin surface after the interstitial fluid is absorbed; and degrading the plurality of microneedles by contacting the plurality of microneedles with a reducing agent, thereby extracting the interstitial fluid from the plurality of microneedles. In some embodiments, the disulfide bond is configured to be cleaved upon exposure to the reducing agent. In some embodiments, the reducing agent is tris(2-carboxyethyl)phosphine (TCEP). In some embodiments, the disulfide bond is configured to be cleaved by exposure to about 1 mM to about 100 mM of TCEP. In some embodiments, the reducing agent is glutathione, dithiothreitol, or beta-mercaptoethanol. In some embodiments, the interstitial fluid comprises a biomarker and/or a cell.

In some embodiments, the biomarker comprises a cytokine, a chemokine, glucose, bilirubin, carnosine, cortisol, creatine, creatinine, homocysteine, uric acid, vitamin A, vitamin B12, lumazine, pantothenic acid (vitamin B5), lumichrome, pyridoxamine (vitamin B6 form), pyridoxal (vitamin B6 form), 4-pyridoxic acid, ascorbic acid (vitamin C), ergocalciferol (vitamin D2), 7-dehydrocholesterol (vitamin D3), hypoxanthine, nicotinamide adenine dinucleotide (NAD), uridine, xanthine, myristic acid (c14:0), palmitoleic acid (c16:1), stearic acid (c18:0), arachidic acid (c20:0), cholic acid, glycocholic acid, urocanic acid, 4-Guanidinobutanoic acid, succinylhomoserine, tocopherol, N6-(delta2-isopentenyl)-adenine, nebularine, cytidine monophosphate (CMP), cytidine, inosine, 3-methyladenine, nicotinamide ribotide, N-methyltryptamine, sphingosine, 20-COOH-leukotriene B4, stachyose, gulonolactone, fructose 6-phosphate, rhamnose, oxalic acid, phosphoenolpyruvic acid, diethanolamine, cyclohexane-1,2-diol, triethanolamine, methyl jasmonate, or any combinations thereof.

Certain aspects of the present disclosure are directed to hydrogel compositions comprising: a degradable hyaluronic acid polymer comprising the following chemical structure: 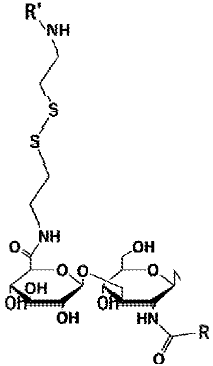 wherein the R group is a methyl group and the R' group is hydrogen.

Certain aspects of the present disclosure are directed to methods of delivering a therapeutic agent to a subject in need thereof, the method comprising: contacting the subject with the hydrogel compositions of the disclosure, the hydrogel configured to be degraded upon exposure to a reducing agent, wherein the hydrogel composition is crosslinked with a crosslinker comprising PEG further comprising a succinimidyl functional group.

In some embodiments, the hydrogel is a microneedle array, an injectable hydrogel, a pre-formed hydrogel depot, a sprayable hydrogel, or any combination thereof.

The terms "subject" or "patient" as used herein refer to any mammal (e.g., a human or a veterinary subject, e.g., a dog, cat, horse, cow, goat, sheep, mouse, rat, or rabbit) to which a composition or method of the present disclosure may be administered, e.g., for experimental, diagnostic, prophylactic, and/or therapeutic purposes. The subject may seek or need treatment, require treatment, is receiving treatment, will receive treatment, or is under care by a trained professional for a particular disease or condition.

The term "composition" as used herein can refer to a microneedle array composition, a precursor composition (e.g., a composition before crosslinking polymerization), and/or a hydrogel composition (e.g., a hydrogel composition after crosslinking polymerization), as provided by the corresponding context of the disclosure.

As used herein, the singular forms "a," "an," and "the" include plural referents unless the context clearly dictates otherwise. Thus, for example, reference to "a micelle" includes mixtures of micelles, reference to "a micelle" includes mixtures of two or more such micelles, and the like.

As used herein, the term "therapeutic agent" is any molecule or atom that is encapsulated, conjugated, fused, dispersed, embedded, mixed, or otherwise affixed to any of the compositions described herein and is useful for a disease therapy.

As used herein, the term "payload" refers to an agent delivered by a chemically-modified hydrogel composition described herein (e.g., chemically-modified hyaluronic acid hydrogel microneedles or other chemically-modified hyaluronic acid hydrogel compositions).

By the term "nanoparticle" is meant an object that has a diameter between about 2 nm to about 200 nm (e.g., between 10 nm and 200 nm, between 2 nm and 100 nm, between 2 nm and 40 nm, between 2 nm and 30 nm, between 2 nm and 20 nm, between 2 nm and 15 nm, between 100 nm and 200 nm, and between 150 nm and 200 nm). Non-limiting examples of nanoparticles include the nanoparticles described herein. Additional examples of nanoparticles are known in the art.

By the term "nucleic acid" is meant any single- or double-stranded polynucleotide (e.g., DNA or RNA having a semi-synthetic or a synthetic origin). The term nucleic acid includes oligonucleotides containing at least one modified nucleotide (e.g., containing a modification in the base and/or a modification in the sugar) and/or a modification in the phosphodiester bond linking two nucleotides. Exemplary nucleic acids for use in accordance with the present disclosure include, but are not limited to, one or more of DNA, RNA, hybrids thereof, RNAi-inducing agents, RNAi agents, siRNAs, shRNAs, miRNAs, mRNAs, antisense RNAs, ribozymes, catalytic DNA, RNAs that induce triple helix formation, aptamers, vectors, and the like. Additional non-limiting examples of nucleic acids are described herein and are known in the art.

As used herein, the expression "pharmaceutically acceptable" applies to a composition that contains composition ingredients that are compatible with other ingredients of the composition as well as physiologically acceptable to the recipient (e.g., a mammal such as a human) without the resulting production of excessive undesirable and unacceptable physiological effects or a deleterious impact on the mammal being administered the pharmaceutical composition. A composition as described herein can comprise one or more carriers, useful excipients, and/or diluents.

As used herein, the term "hydrogel" refers to a polymeric material having a three-dimensional physical or covalently cross-linked networks that have an affinity for an aqueous medium and are able to absorb a large amount of water while maintaining a semisolid morphology (e.g., they do not normally dissolve in the aqueous medium unless they are triggered to do so).

As used herein, the term "aqueous medium" as used herein refers to water or a solution based primarily on water such as phosphate-buffered saline (PBS), or water containing one or more salts dissolved therein.

As used herein, the term "crosslink" refers to an interconnection between polymer chains via chemical bonding, such as, but not limited to, covalent bonding, ionic bonding, or affinity interactions that are caused by a chemical composition (e.g., a crosslinker).

As used herein, the term "biodegradable" refers to a substance which may be broken down by microorganisms, or which spontaneously breaks down over a relatively short time (within about 14 days to about 6 months) when exposed to environmental conditions commonly found in nature. For example, the compositions described herein may be degraded by a reducing agent (e.g., TCEP) that is contacted with the composition.

Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. Furthermore, the use of the term "about," as used herein, refers to an amount that is near the stated amount by 10%, 5%, or 1%, including increments therein. In the current description "about" means a range including the particular value and ranging from 10% below that particular value and spanning to 10% above that particular value.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features.

Where values are described in the present disclosure in terms of ranges, endpoints are included. Furthermore, it should be understood that the description includes the disclosure of all possible sub-ranges within such ranges, as well as specific numerical values that fall within such ranges irrespective of whether a specific numerical value or specific sub-range is expressly stated.

Various embodiments of the features of this disclosure are described herein. However, it should be understood that such embodiments are provided merely by way of example, and numerous variations, changes, and substitutions can occur according to those skilled in the art without departing from the scope as defined by the claims.

Unless otherwise defined, all technical and scientific terms used herein have the same meaning as commonly understood by one of ordinary skill in the art to which this invention belongs. Methods and materials are described herein are illustrative only and not intended to be limiting. In case of conflict, the present specification, including definitions, will control.

The details of one or more embodiments of the invention are set forth in the accompanying drawings and the description below. Other features and advantages of the invention will be apparent from the following detailed description and figures, and from the claims.

### DESCRIPTION OF DRAWINGS

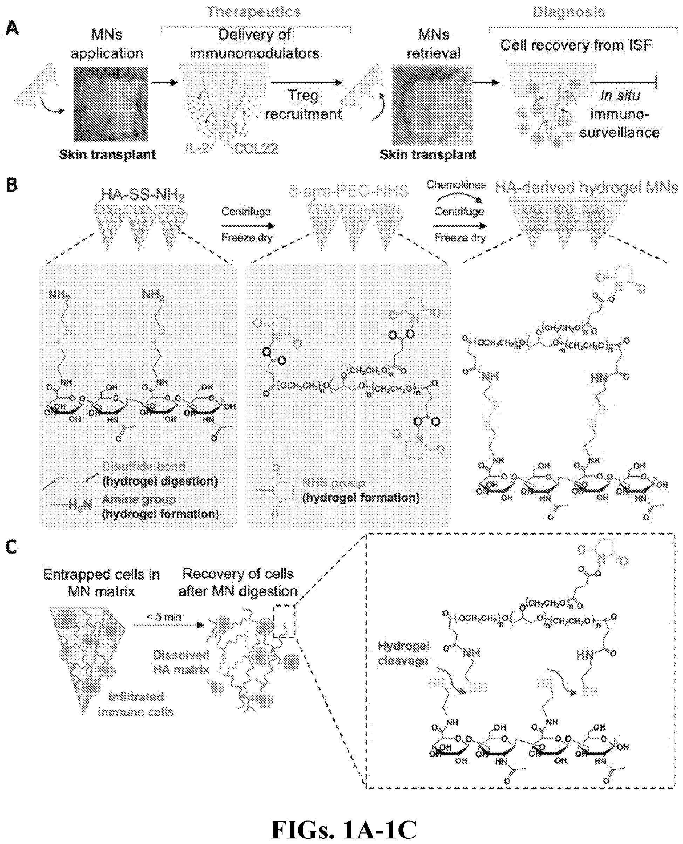

**FIGs. 1A-1C** show the design of an HA-based MN platform. **FIG. 1A** illustrates the use of a novel HA-based MN platform that allows for: (1) delivery of drugs and (2) *in situ* biomarkers and cell sampling for monitoring. **FIG. 1B** illustrates the HA-based MN fabrication process. HA-based MNs were fabricated by casting an aqueous amine-modified HA (HA-SS-NH₂) solution into the PDMS mold by centrifugation and crosslinked using the NHS-terminated 8-arm PEG crosslinker. Chemokines were loaded and a PLGA back layer was added (top scheme). Chemical structure of the HA-SS-NH₂ crosslinked with NHS-terminated 8-arm PEG forming a digestible HA hydrogel through a disulfide bond (bottom scheme). **FIG. 1C** illustrates how the degradation of the MNs under reducing conditions enables facile recovery of retrieved cells. Disulfide bonds of the HA-based MNs are cleaved with 10 mM TCEP.

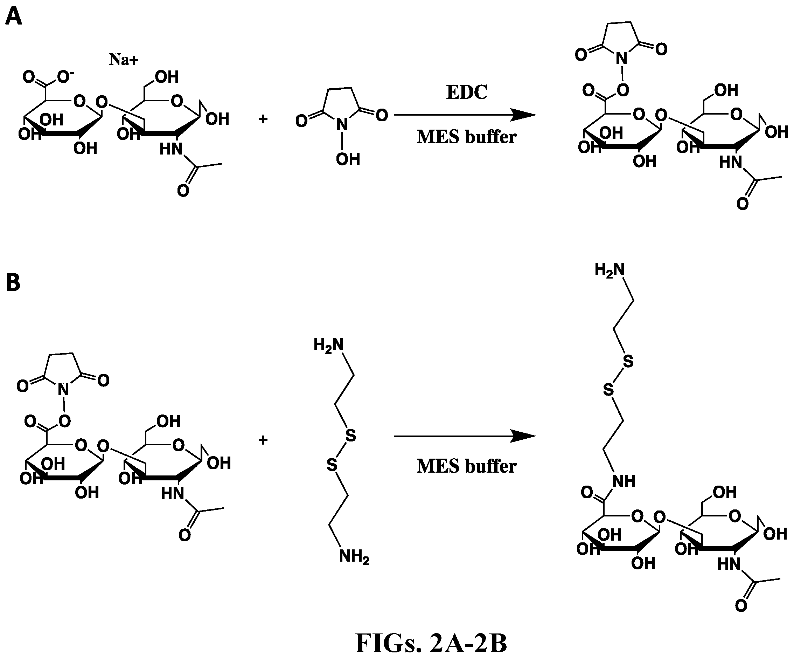

**FIGs. 2A-2B** show the synthesis of HA-SS-NH2 polymer. **FIG. 2A** is a schematic illustrating how 60 kDa-sodium hyaluronate was activated with N-(3-(dimethylamino)propyl)carbodiimide (EDC) and N-hydroxysuccinimide (NHS) at a 1:4:2 molar ratio. **FIG. 2B** is a schematic illustrating how the activated hyaluronic acid is then mixed with Cysteamine Dihydrochloride at a 1:10 molar ratio.

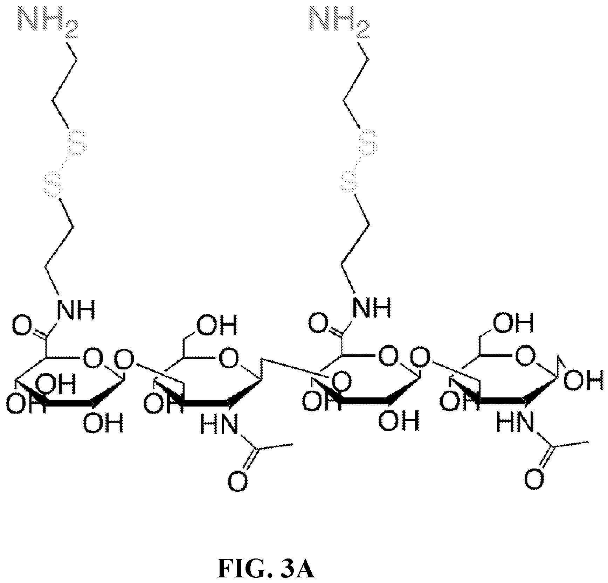

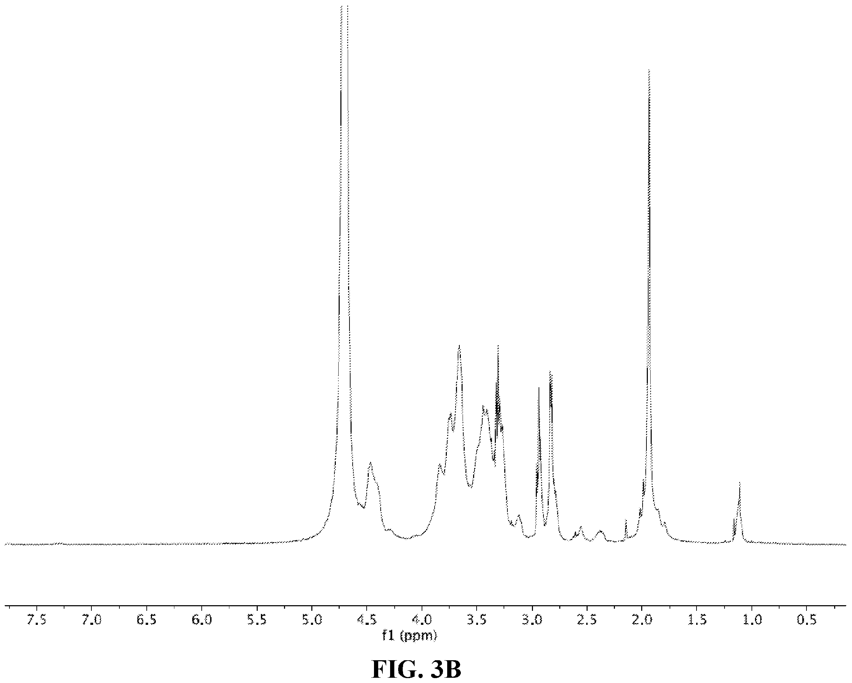

**FIG. 3A** is the chemical structure of the HA-SS-NH₂ polymer. **FIG. 3B** is the ¹H-NMR of the HA-SS-NH₂ polymer.

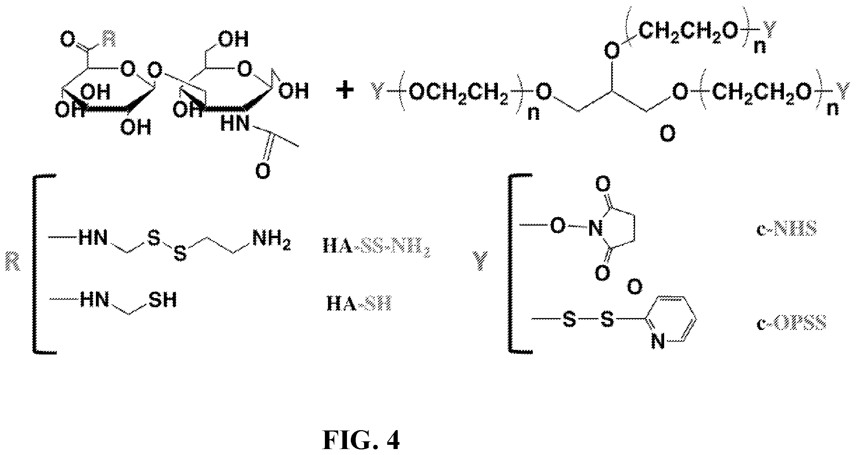

**FIG. 4** shows examples of chemistries that can be used to fabricate HA-based MNs with subsequent on-demand digestion under reducing conditions. The addition of a reductive agent cleaves the disulfide bond present in either the polymer backbone or the crosslinker.

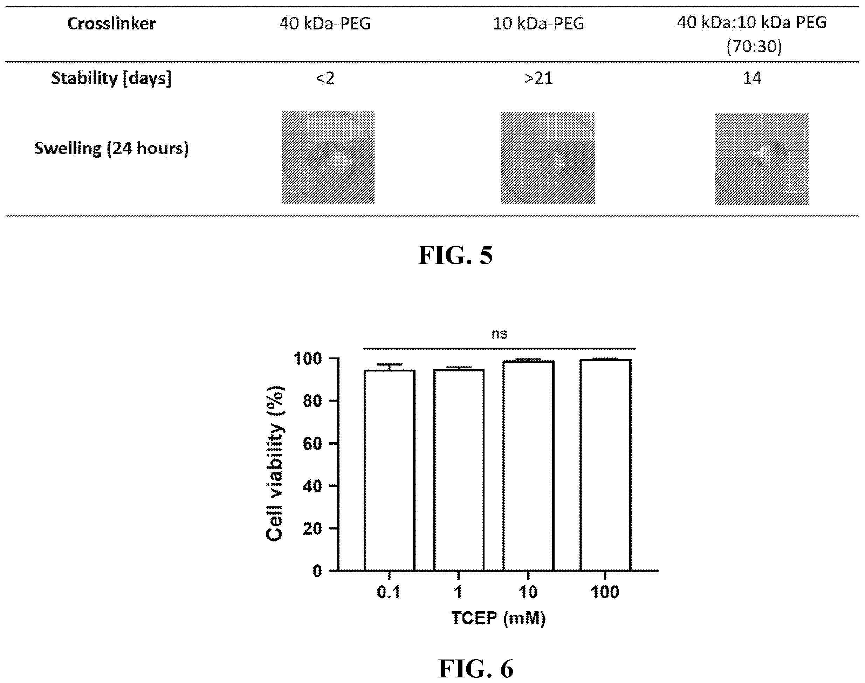

**FIG. 5** shows the stability and photographic images of the various hydrogel disk formulations when incubated in phosphate buffer at 37 °C under rotation.
**FIG. 6** shows cell viability percentages of THP-1 cells following treatment with increasing concentrations of TCEP. Cell death was analyzed by flow cytometry after staining using the LIVE/DEAD fixable Violet Dead Cell Stain. Multiple comparisons among groups were determined using one-way ANOVA followed by a post-hoc test. P-value: *p < 0.05, **p < 0.01.

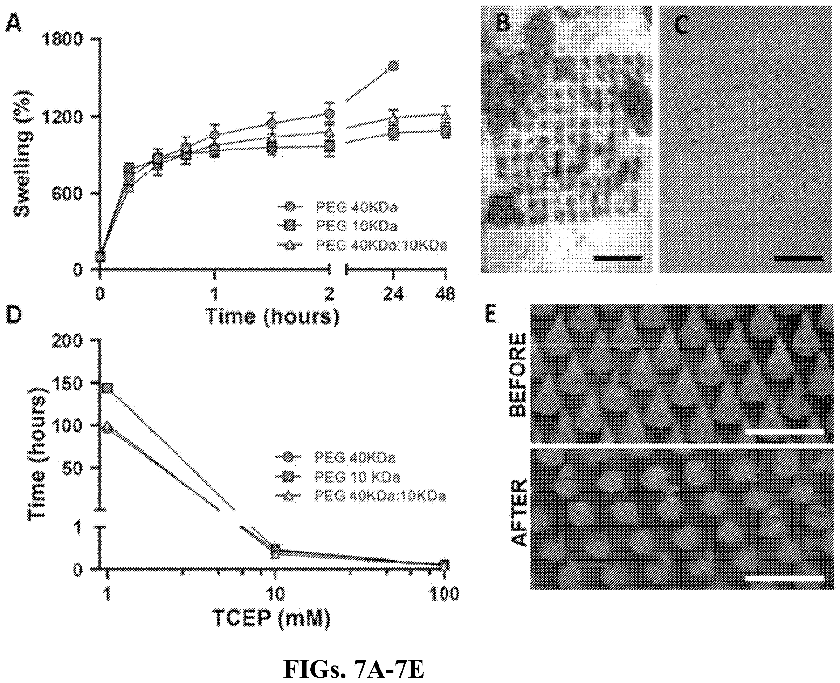

**FIGs. 7A-7E** show HA-modified MNs present high swelling capacity, robust mechanical properties and on-demand degradation. **FIG. 7A** is a graph showing the swelling rate of the HA-based hydrogels composed of amine-modified HA polymer crosslinked with NHS-terminated 8-arm PEG crosslinkers differing in molecular weight. **FIG. 7B** is an image of stained skin graft following *ex vivo* HA-SS-NH₂-derived MN application confirms skin penetration. Scale bar = 2mm. **FIG. 7C** is an image showing the mouse skin after *in vivo* administration of hydrogel MNs. Scale bar = 2mm. **FIG. 7D** is a graph showing on-demand digestion of hydrogel disks using varying concentrations of the reducing agent, TCEP. **FIG. 7E** is a group of optical microscopy images of hydrogel-based MNs before (top) and after (bottom) digestion under reducing conditions. Scale bar = 500µm. Data is represented as mean ± s.d. (n = 3).

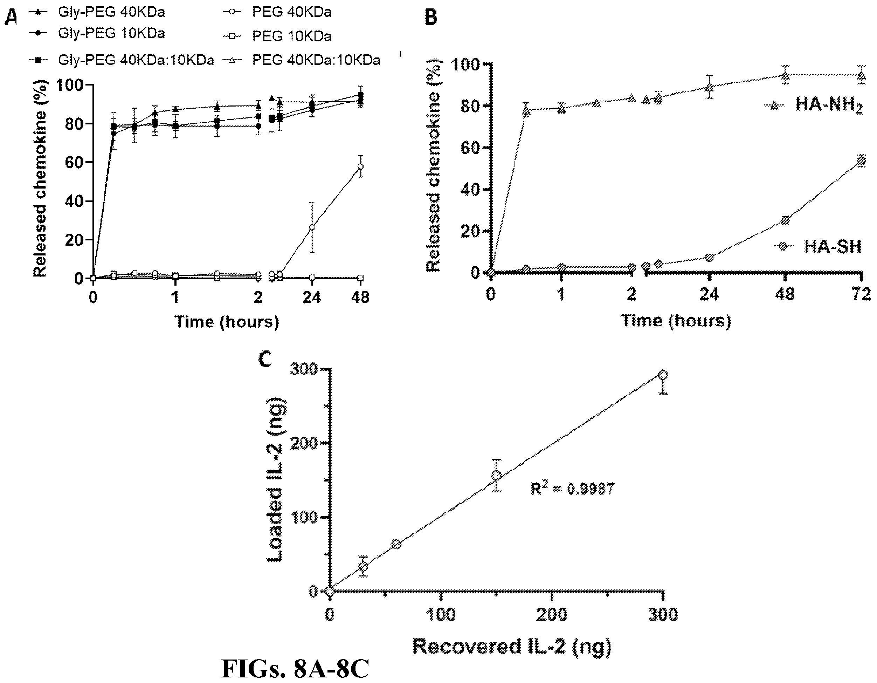

**FIGs. 8A-8C** show the study of the therapeutic potential of the HA-derived MNs. **FIG. 8A** is a graph showing the release of the IL-2 (drug model) from the HA-based hydrogel MNs quantified by tracking the fluorescence signal of the labeled IL-2 over time. **FIG. 8B** is a graph showing the comparison of the release profile between MNs derived from the HA-SH polymer and the HA-NH2. **FIG. 8C** is a graph showing the analysis of the MNs loading capacity by means of fluorescence quantification (plotted as initially loaded mass of IL-2 versus recovered mass of IL-2, R2 = 0.9987).

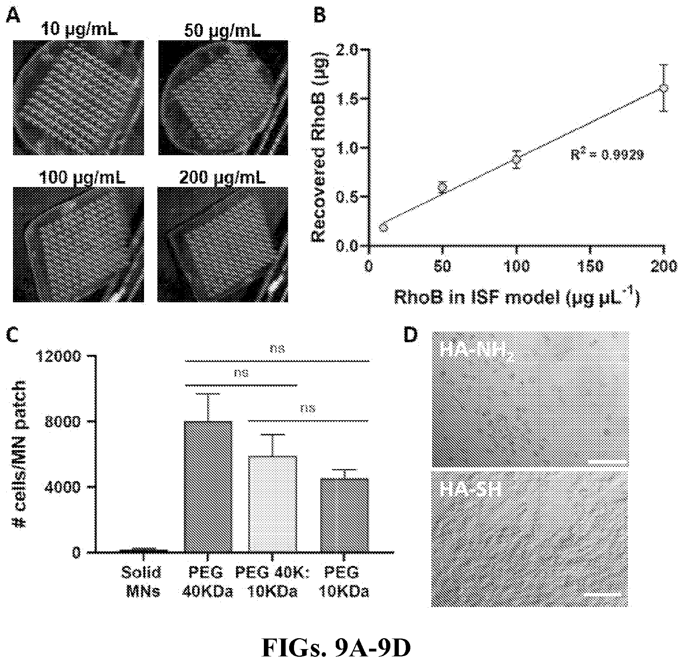

**FIGs. 9A-9D** shows the characterization of MN-based platform for immune cell sampling. **FIG. 9A** is a set of images showing the analyte recovery (RhoB) from mimetic skins containing increasing amounts of RhoB with HA-derived MNs. **FIG. 9B** is a graph showing detected RhoB concentration compared to the real RhoB concentration in HA-based MNs, R² = 0.9929. **FIG. 9C** is a graph showing the recovered immune cells from digested HA-based MNs as quantified by flow cytometry. Data is represented as mean ± s.d. (n = 3). Multiple comparisons among groups were determined. **FIG. 9D** is a group of microscopic images of HA-derived hydrogels functionalized with different pendants groups showing cell infiltration. Hydrogels were incubated with THP-1 cells and imaged 24 hours post-incubation.

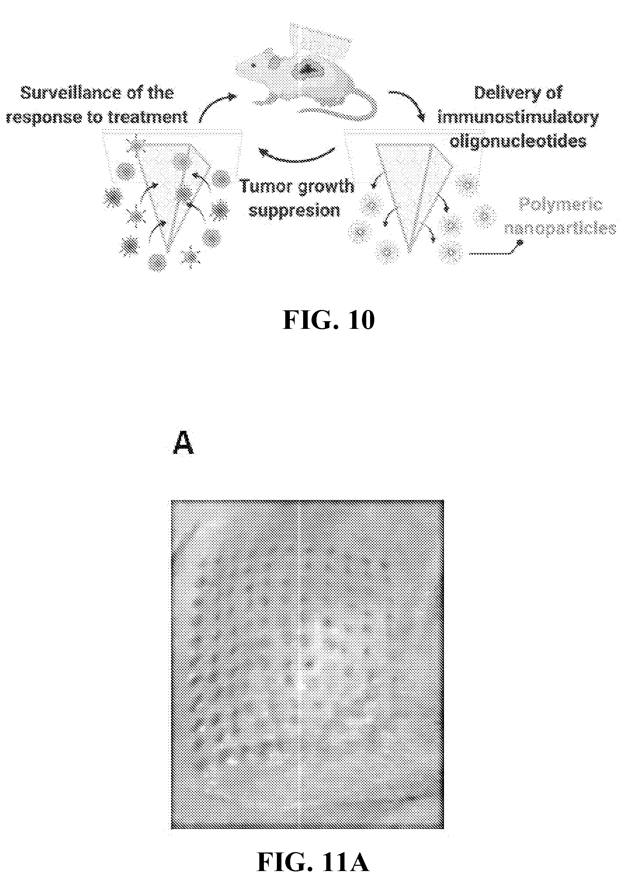

**FIG. 10** is a schematic illustrating how hydrogel-based MNs can deliver immunostimulatory therapies to restrict tumor growth while surveilling simultaneously the immune cell profile as a response to therapy.

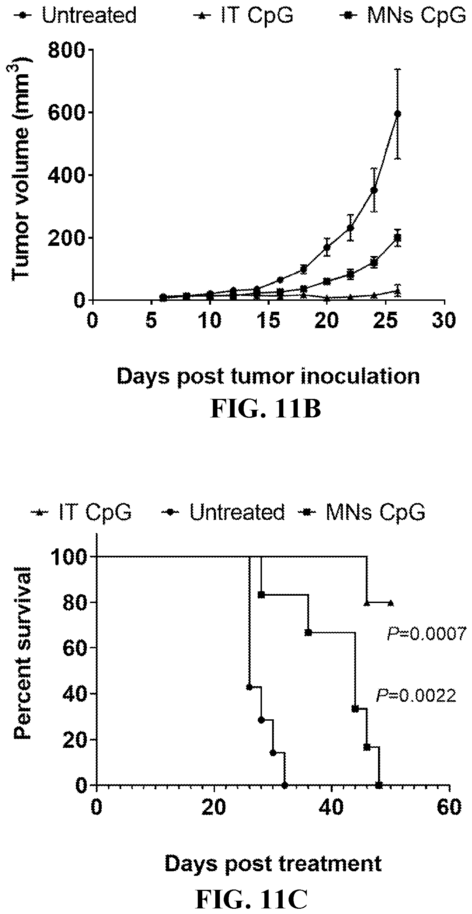

**FIG. 11A** is an image showing the skin disruption of the area surrounding the subcutaneous tumor following MN administration. **FIG. 11B** is a graph showing the representation of caliper measurements of the tumor volume after consecutive administration of CpG-containing nanoparticles (1 µg /dose) via MN delivery of intratumoral injection. **FIG. 11C** is a graph showing the representation of mice survival after completion of the treatment.

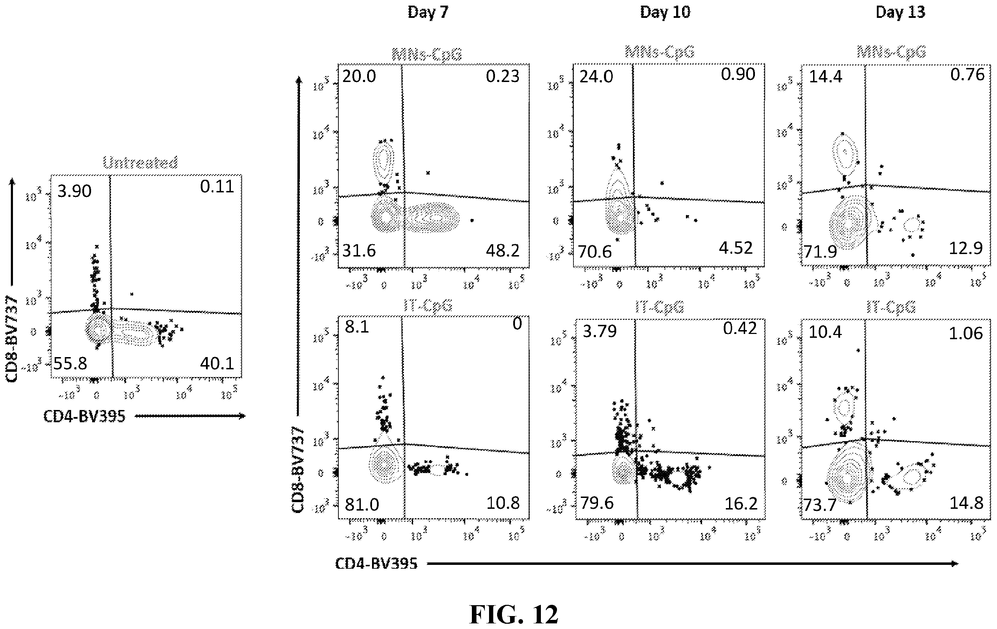

**FIG. 12** shows immunophenotyping of the ISF surrounding the tumor microenvironment using HA-based MNs. MNs were applied 24 hours on the tumor area to maximize the collection of immune cells. Next, MN patches were digested on-demand under reducing conditions and the isolated cells were analyzed by flow cytometry. The immune profile in the tumor microenvironment was monitored to study the shifting in immune populations as a response to therapy.

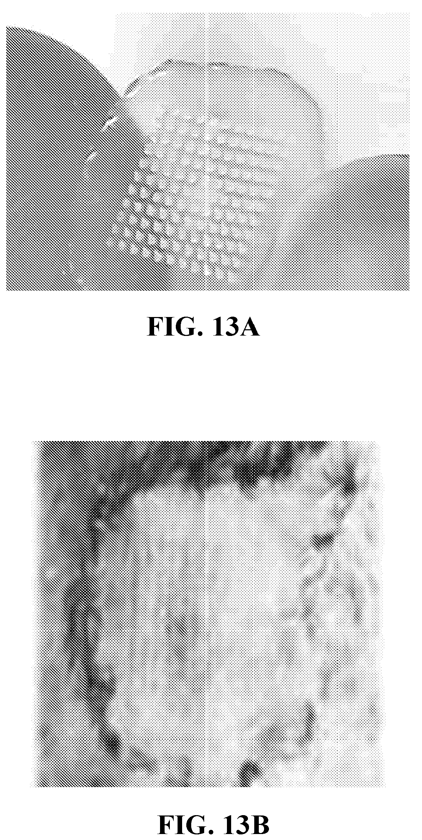

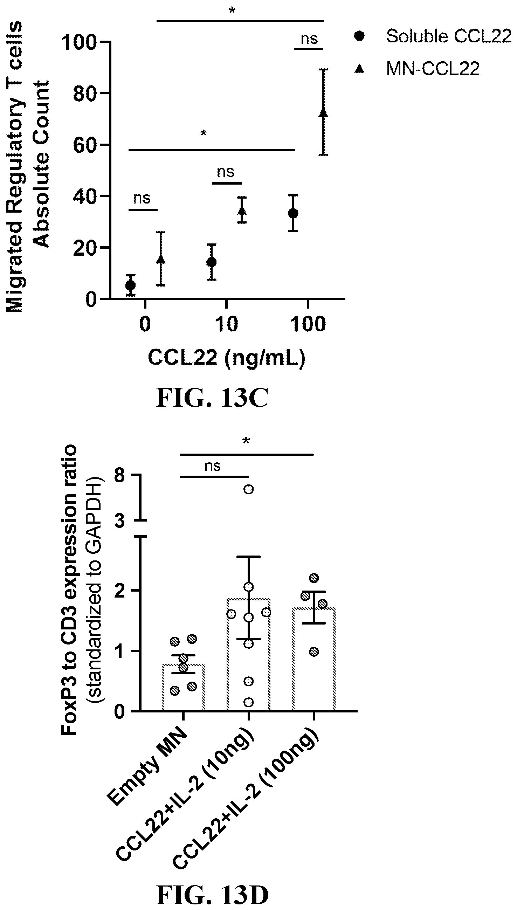

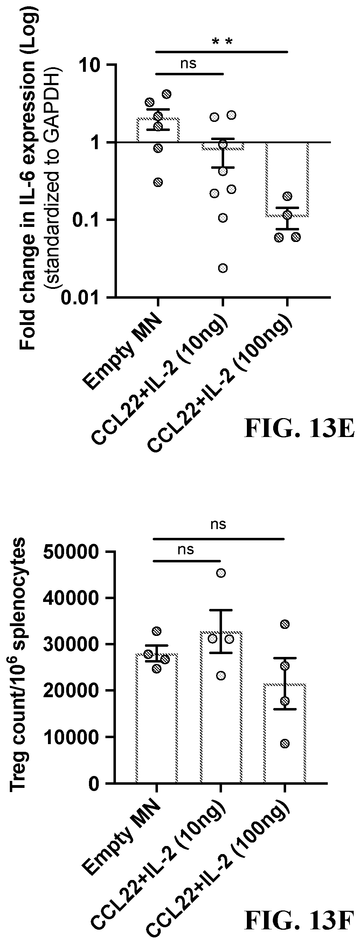

**FIG. 13A** is an image showing the MN patch (1cm²). **FIG. 13B** is an image of a skin allograft after *in vivo* administration of the MNs showing effective penetration. A skin patch from BALB/c was transplanted onto the dorsal trunk of a Rag-/- mouse, followed by the treatment consisting in the administration of CCL22+IL-2 loaded MNs for 5 days consecutive days. **FIG. 13C** is a graph showing the migrated regulatory T cells as a function of CCL22. At day 7, skin was harvested and analyzed by RT-PCR. **FIG. 13D** is a graph showing the FOXP3 to CD3 expression ratio. **FIG. 13E** is a graph showing the fold change in IL-6 gene expression. **FIG. 13F** is a graph showing quantification by flow cytometry of the number of Tregs (FOXP3+; CD4+) per million of splenocytes following CCL22+IL-2 administration compared to empty MNs as control.

### DETAILED DESCRIPTION

The compositions described herein include biocompatible, chemically crosslinkable hyaluronic acid-based hydrogels including microneedle arrays. In some examples, the compositions described herein are used for targeted, transdermal drug delivery. Methods of using and/or preparing these compositions are also provided herein. Some embodiments of the compositions and methods described herein may provide one or more of the following advantages.

Certain embodiments of the present disclosure include biocompatible, crosslinkable hyaluronic acid-based hydrogels. As discussed above, there is currently an unmet need for a capable of therapeutic and diagnostic applications. The compositions and methods of the present disclosure address this need. For example, in some embodiments, the microneedle array compositions and methods described herein can deliver a therapeutic agent payload while simultaneously sampling an interstitial fluid in a tissue, thereby offering an opportunity for diagnosis and monitoring of the response to therapy to further personalize it to the needs of the patient. In some embodiments, the chemical structure of the biocompatible polymer together with a crosslinker forms highly swellable hydrogel microneedles. In some embodiments, upon retrieval, the microneedles can be digested ex vivo by adding a reductive agent in less than 5 minutes for subsequent recovery and analysis of the biomarkers. Hence, the microneedle array compositions disclosed herein are a platform that can provide a quick readout of the patient state to intercept the disease and treat it prior to reaching irreversible states.

Some embodiments described herein may provide a theranostic microneedle platform using hyaluronic acid to deliver different types of drugs while enabling simultaneous sampling of the soluble and cellular fraction of the ISF. In some embodiments, the HA-based microneedles offer provide non-invasive delivery of various therapeutic cargos while retrieving biomarkers present in ISF. In some embodiments, the various therapeutic cargoes include a molecules of a wide range of molecular weights in the nano- and micro-scales, as outlined in detail elsewhere herein.

In some embodiments, the microneedle-based delivery methods described herein allow precise local delivery of a therapeutic agent within the skin, thereby reducing the off-target, side effects associated with systemic drug delivery. In some embodiments, the microneedle array compositions and methods of the disclosure are non-invasive and pain-free, thereby facilitating high patient compliance while minimizing the risk of infections and needle-borne diseases. In some embodiments, the microneedle array compositions and methods of the disclosure can be used for diagnostic purposes serving as a non-invasive tool for interstitial fluid (ISF) extraction. ISF is a rich source of biomarkers that has been confirmed in recent clinical trials to intimately correlate with those biomarkers present in plasma and other conventional sources. Therefore, in some embodiments, ISF monitoring using the microneedle array compositions and methods of the disclosure can inform on tissue physiology by sampling both soluble biomarkers and cells and in turn report on the patient physiological state.

Some embodiments described herein may provide a hydrogel composition that may have tunable properties. For example, the swelling ratios, mechanical strength, degradation, and drug release profiles of the hydrogel compositions described herein may be optimized by varying the molecular weight and concentration of one or more crosslinkers. Furthermore, in some embodiments, the hydrogel compositions described herein have on-demand degradation that is controlled by exposure to a reducing agent. Thus, the in some embodiments, the compositions and methods of the disclosure may provide a flexible drug delivery platform that can be optimized for various applications.

### Microneedle (MN) Arrays

### Structure of MN Arrays

The present disclosure features microneedle array compositions that can include a plurality of microneedles projecting from a substrate. In some embodiments, the plurality of microneedles of the disclosure are degradable, porous, drug-eluting hydrogels that may facilitate the delivery of a drug through the structural barriers of tissues (e.g., skin) and/or sampling of interstitial fluid within a tissue (e.g., a skin tissue) upon application, as illustrated in **FIG. 1A****.** For example, the microneedle arrays of the disclosure can be applied to a skin surface to deliver a therapeutic agent directly to an injured site, a disease site, and/or a transplant site in the skin of a patient. To this end, the microneedle array compositions described herein can further include one or more therapeutic agents.

In some embodiments, the microneedle array comprises a substrate from which the plurality of microneedles project therefrom. In some embodiments, the substrate is configured to be an anchor for microneedle array administration and retrieval. For example, when in use, the user (e.g., a clinician) can grasp the microneedle array by a substrate surface or an edge of the surface prior to applying the microneedle array on a skin surface of the patient. In some embodiments, the substrate is a substantially planar surface. In some embodiments, the substrate is a backing layer. In some embodiments, the substrate is a polymeric substrate. In some embodiments, the substrate is a biodegradable substrate. In some embodiments, the substrate is a non-biodegradable substrate. In some embodiments, the substrate comprises a non-soluble polymer. In some embodiments, the substrate comprises poly(D,L-lactide-co-glycolide) (PLGA) polymer. In some embodiments, the substrate comprises a water-soluble polymer. In some embodiments, the substrate comprises poly(vinyl alcohol) (PVA). In some embodiments, the substrate comprises polyethylene glycol diacrylate. Non-limiting examples of polymers that can be used to prepare a substrate include poly(caprolactone), poly(ethylene) glycol, poly(vinyl) pyrrolidone, poly(2-hydroxy ethyl methacrylate), poly(N-vinyl pyrrolidone), poly(methyl methacrylate), poly(vinyl alcohol), poly(acrylic acid), polyacrylamide, poly(ethylene-co-vinyl acetate), poly(ethylene glycol), poly(methacrylic acid), polylactides (PLA), polyglycolides (PGA), polyanhydrides, polyorthoesters, polycyanoacrylate polycaprolactone, cellulose, lignin, alginate, chitosan, starch, or any combination thereof.

In some embodiments, each microneedle of the plurality of microneedles comprises a penetrating tip and a base that is integrally connected with the substrate. In some embodiments, each microneedle of the plurality of microneedles comprises a penetrating tip and a base that is removably connected with the substrate (e.g., each microneedle can be configured to be detached from the substrate via a trigger mechanism such as the dissolution of the substrate). In some embodiments, each microneedle has an elongate body having a proximal end and a distal end. In some embodiments, the elongate body generally tapers from the proximal end, near the base, to the distal end, near the penetrating tip. In some embodiments, each microneedle has a pyramidal or conical shape such that the microneedles taper to a point or a tip that is configured to perforate and/or penetrate a skin surface. The dimensions and geometry of the microneedles can vary as desired.

In some embodiments, each microneedle has a height ranging from about 100 µm to about 1,500 µm (e.g., about 100 µm to about 600 µm, about 200 µm to about 600 µm, about 300 µm to about 600 µm, about 400 µm to about 600 µm, about 500 µm to about 600 µm, about 600 µm to about 700 µm, about 600 µm to about 800 µm, about 600 µm to about 900 µm, about 600 µm to about 1000 µm, about 600 µm to about 1100 µm, about 600 µm to about 1200 µm, about 600 µm to about 1300 µm, about 600 µm to about 1400 µm, or about 600 µm to about 1500 µm). In some embodiments, each microneedle has a height of about 600 µm. The height of each microneedle can be measured from the base at the proximal end of the microneedle to the tip at the distal end of the microneedle. In some embodiments, each microneedle has a height that is sufficient to penetrate the stratum corneum and pass into the epidermis and/or the dermis.

In some embodiments, each microneedle has a base (e.g., a circular base or a rectangular base) having a width (e.g., a radius or rectangular width) ranging from about 100 µm to about 10,000 µm (e.g., about 100 µm to about 150 µm, about 125 µm to about 150 µm, about 150 µm to about 200 µm, about 150 µm to about 300 µm, about 150 µm to about 400 µm, about 150 µm to about 500 µm, about 150 µm to about 600 µm, about 150 µm to about 700 µm, about 150 µm to about 800 µm, about 150 µm to about 900 µm, about 150 µm to about 1000 µm, about 150 µm to about 1100 µm, about 150 µm to about 1200 µm, about 150 µm to about 1300 µm, about 150 µm to about 1400 µm, about 150 µm to about 1500 µm, about 150 µm to about 5000 µm, about 150 µm to about 7,500 µm, about 150 µm to about 10,000 µm, about 1500 µm to about 5000 µm, about 1500 µm to about 7,500 µm, or about 1500 µm to about 10,000 µm). In some embodiments, each microneedle has a circular base having a radius of about 150 µm. In some embodiments, each microneedle has a rectangular base having a width of about 300 µm. The radius of a circular base of each microneedle can be defined as the distance from the center of the circular base to an edge of the circular base. The width of a rectangular base of each microneedle can be defined as the distance from a first edge of the rectangular base to a second, directly opposing edge of the rectangular base.

In some embodiments, each microneedle has a tip width ranging from about 1 µm to about 500 µm (e.g., about 1 µm to about 5 µm, about 1 µm to about 10 µm, about 1 µm to about 15 µm, about 1 µm to about 20 µm, about 1 µm to about 25 µm, about 1 µm to about 30 µm, about 5 µm to about 10 µm, about 5 µm to about 15 µm, about 5 µm to about 20 µm, about 5 µm to about 25 µm, about 5 µm to about 30 µm, about 10 µm to about 15 µm, about 10 µm to about 20 µm, about 10 µm to about 25 µm, about 10 µm to about 30 µm, 15 µm to about 20 µm, about 15 µm to about 25 µm, about 15 µm to about 30 µm, about 20 µm to about 25 µm, about 20 µm to about 30 µm, about 25 µm to about 30 µm, about 1 µm to about 300 µm, about 1 µm to about 400 µm, about 1 µm to about 500 µm, about 300 µm to about 400 µm, about 300 µm to about 500 µm, about 10 µm to about 300 µm, about 50 µm to about 300 µm, about 100 µm to about 300 µm, about 150 µm to about 300 µm, or about 250 µm to about 300 µm). In some embodiments, each microneedle has a tip width of about 300µm.

In some embodiments, the microneedle array can have any suitable shape or size. **In** some embodiments, the microneedle array is an array of microneedles having dimensions ranging from about 5 x 5 to about 20 x 20 (e.g., about 5 x 5 to about 11 x 11, about 6 x 6 to about 11 x 11, about 7 x 7 to about 11 x 11, about 8 x 8 to about 11 x 11, about 9 x 9 to about 11 x 11, about 10 x 10 to about 11 x 11, about 11 x 11 to about 15 x 15, about 11 x 11 to about 20 x 20. In some embodiments, the microneedle array is an 11 x 11 array of microneedles. In some embodiments, a density of the plurality of microneedles of the microneedle array can range between about 100 microneedles/cm² to about 1000 microneedles/cm² or more (e.g., about 100 microneedles/cm² to about 500 microneedles/cm², about 100 microneedles/cm² to about 600 microneedles/cm², about 100 microneedles/cm² to about 700 microneedles/cm², about 100 microneedles/cm² to about 800 microneedles/cm², about 900 microneedles/cm² to about 500 microneedles/cm², about 100 microneedles/cm² to about 950 microneedles/cm², about 500 microneedles/cm² to about 600 microneedles/cm², about 500 microneedles/cm² to about 700 microneedles/cm², about 500 microneedles/cm² to about 800 microneedles/cm², about 500 microneedles/cm² to about 900 microneedles/cm², about 500 microneedles/cm² to about 1000 microneedles/cm², or more)._In some embodiments, a density of the plurality of microneedles of the microneedle array is about 500 microneedles/cm².

In some embodiments, the microneedle array can be arranged in a variety of ways. In some embodiments, the microneedle array can be arranged with a tip-to-tip spacing between microneedles ranging from about 50 µm to about 1000 µm (e.g., about 50 µm to about 600 µm, about 100 µm to about 600 µm, about 200 µm to about 600 µm, about 300 µm to about 600 µm, about 400 µm to about 600 µm, about 500 µm to about 600 µm, about 600 µm to about 700 µm, about 600 µm to about 800 µm, about 600 µm to about 900 µm, or about 600 µm to about 1000 µm). In some embodiments, the microneedle array can be arranged with a tip-to-tip spacing between microneedles of about 600 µm.

### Functionalized HA Polymer

Hyaluronic acid (HA) is a viscoelastic, biocompatible, biodegradable, non-toxic, and non-immunogenic natural linear polysaccharide with high water affinity. HA is known to play a role in the regeneration and reconstruction of soft tissues. In some embodiments, a chemically-modified HA can be included in the microneedle array compositions of the present disclosure. In some embodiments, the entire structure of each microneedle (e.g., from the base to the tip) is composed of the chemically-modified HA described herein.

The chemically modified HA is an amino-modified hyaluronic acid comprising a disulfide bond (HA-SS-NH₂).

In some embodiments, the HA polymer that is chemically modified has a molecular weight ranging from about 0.5 kilodalton (kDa) to about 20,000 kDa (e.g., about 0.5 kDa to about 60 kDa, about 1 kDa to about 60 kDa, about 5 kDa to about 60 kDa, about 10 kDa to about 60 kDa, about 20 kDa to about 60 kDa, about 30 kDa to about 60 kDa, about 40 kDa to about 60 kDa, about 50 kDa to about 60 kDa, about 60 kDa to about 75 kDa, about 60 kDa to about 100 kDa, about 60 kDa to about 200 kDa, about 60 kDa to about 300 kDa, about 60 kDa to about 400 kDa, about 60 kDa to about 500 kDa, about 60 kDa to about 600 kDa, about 60 kDa to about 700 kDa, about 60 kDa to about 800 kDa, about 60 kDa to about 900 kDa, about 60 kDa to about 1000 kDa, about 60 kDa to about 2000 kDa, about 60 kDa to about 3000 kDa, about 60 kDa to about 4000 kDa, about 60 kDa to about 5000 kDa, about 60 kDa to about 6000 kDa, about 60 kDa to about 7000 kDa, about 60 kDa to about 8000 kDa, about 60 kDa to about 9000 kDa, about 60 kDa to about 10,000 kDa, about 60 kDa to about 11,000 kDa, about 60 kDa to about 12,000 kDa, about 60 kDa to about 13,000 kDa, about 60 kDa to about 14,000 kDa, about 60 kDa to about 15,000 kDa, about 60 kDa to about 16,000 kDa, about 60 kDa to about 17,000 kDa, about 60 kDa to about 18,000 kDa, about 60 kDa to about 19,000 kDa, about 60 kDa to about 20,000 kDa, about 1000 kDa to about 5000 kDa, about 1000 kDa to about 10,000 kDa, about 1000 kDa to about 15,000 kDa, about 1000 kDa to about 20,000 kDa.

In some embodiments, the chemically-modified HA polymer includes one or more side chains including one or more functional groups (e.g., a disulfide group and an amine group), each side chain having a length ranging from about 3 carbon atoms to about 100 carbon atoms (e.g., about 3 carbon atoms to about 4 carbon atoms, about 3 carbon atoms to about 5 carbon atoms, about 3 carbon atoms to about 6 carbon atoms, about 3 carbon atoms to about 7 carbon atoms, about 3 carbon atoms to about 8 carbon atoms, about 3 carbon atoms to about 9 carbon atoms, about 3 carbon atoms to about 10 carbon atoms, about 3 carbon atoms to about 15 carbon atoms, about 3 carbon atoms to about 20 carbon atoms, about 3 carbon atoms to about 25 carbon atoms, about 3 carbon atoms to about 30 carbon atoms, about 3 carbon atoms to about 35 carbon atoms, about 3 carbon atoms to about 40 carbon atoms, about 3 carbon atoms to about 45 carbon atoms, about 3 carbon atoms to about 50 carbon atoms, about 3 carbon atoms to about 55 carbon atoms, about 3 carbon atoms to about 60 carbon atoms, about 3 carbon atoms to about 65 carbon atoms, about 3 carbon atoms to about 70 carbon atoms, about 3 carbon atoms to about 75 carbon atoms, about 3 carbon atoms to about 80 carbon atoms, about 3 carbon atoms to about 85 carbon atoms, about 3 carbon atoms to about 90 carbon atoms, about 3 carbon atoms to about 95 carbon atoms, about 3 carbon atoms to about 99 carbon atoms, about 10 carbon atoms to about 50 carbon atoms, about 50 carbon atoms to about 100 carbon atoms, or more).

In some embodiments, the chemically-modified HA polymer includes one or more side chains having a same chain length. In some embodiments, the chemically-modified HA polymer includes one or more side chains having a different chain length. In some embodiments, the one or more side chains include an amine group and a disulfide group. In some embodiments, the one or more side chains include an amine group. In some embodiments, the one or more side chains include a disulfide group. In some embodiments, the chemically modified hyaluronic acid comprises one or more side functional groups. In some embodiments, the chemically modified hyaluronic acid comprises one or more side chains including one or more side functional groups. In some embodiments, the side functional groups comprise one or more disulfide groups, thiol groups, urea groups, carboxylic ester groups, carboxylic acid groups, carboxylic acid salts, latent carboxylic acid groups, quaternary amine groups, tertiary amine groups, secondary amine groups, primary amine groups, azides, alkynes, poly(alkylene ether) groups, and any combinations thereof.

In some embodiments, the plurality of microneedles comprises a degradable hyaluronic acid polymer comprising a disulfide bond. In some embodiments, the degradable hyaluronic acid polymer is cross-linkable. In some embodiments, the degradable hyaluronic acid polymer is an amino-modified hyaluronic acid prior to being crosslinked (e.g., in a precursor state). As illustrated in FIG. 2A and described in Example 1, the hyaluronic acid polymer can be synthesized by activating sodium hyaluronate with N-(3-(dimethylamino)propyl)carbodiimide (EDC) and N-hydroxysuccinimide (NHS). Next, as illustrated in FIG. 2B and also described in Example 1, the activated hyaluronic acid can be mixed with cysteamine dihydrochloride and reacted for about 12 hours. In some embodiments, the activated hyaluronic acid can be mixed with cysteamine dihydrochloride at a ratio ranging from about 1:1 to about 1:20 (e.g., about 1:1 to about 1:10, about 1:2 to about 1:10, about 1:3 to about 1:10, about 1:4 to about 1:10, about 1:5 to about 1:10, about 1:6 to about 1:10, about 1:7 to about 1:10, about 1:8 to about 1:10, about 1:9 to about 1:10, about 1:10 to about 1:15, or about 1:10 to about 1:20). In some embodiments, the activated hyaluronic acid can be mixed with excess cysteamine dihydrochloride. The addition of cysteamine dihydrochloride to the activated hyaluronic acid functionalizes the hyaluronic acid with a terminal amino group and the disulfide bond, as shown in **FIG. 3A****.**

In some embodiments, the plurality of microneedles comprises a degradable hyaluronic acid polymer having an on-demand degradation dependent upon the cleavage of the disulfide bond. For example, in some embodiments, the degradable hyaluronic acid polymer can have a degradation profile that is controlled by the addition of a reducing agent that cleaves the disulfide bond. In some embodiments, each microneedle of the plurality of microneedles is a degradable microneedle configured to be degraded upon exposure to the reducing agent. In some embodiments, the disulfide bond is configured to be cleaved upon exposure to the reducing agent. In some embodiments, the reducing agent is tris(2-carboxyethyl)phosphine (TCEP). In some embodiments, the reducing agent is glutathione, dithiothreitol, beta-mercaptoethanol, or any combination thereof.

In some embodiments, the disulfide bond is configured to be cleaved by exposure to about 1 millimolar (mM) to about 100 mM of TCEP (e.g., about 1 mM to about 10 mM, about 2 mM to about 10 mM, about 3 mM to about 10 mM, about 4 mM to about 10 mM, about 5 mM to about 10 mM, about 6 mM to about 10 mM, about 7 mM to about 10 mM, about 8 mM to about 10 mM, about 9 mM to about 10 mM, about 10 mM to about 20 mM, about 10 mM to about 30 mM, about 10 mM to about 40 mM, about 10 mM to about 50 mM, about 10 mM to about 60 mM, about 10 mM to about 70 mM, about 10 mM to about 80 mM, about 10 mM to about 90 mM, about 10 mM to about 100 mM). In some embodiments, the disulfide bond is configured to be cleaved by exposure to about 10 mM of TCEP.

Generally, a hydrogel may be formed by using at least one, or one or more types of hydrogel precursors, and setting or solidifying the one or more types of hydrogel precursors in an aqueous solution to form a three-dimensional network, wherein formation of the three-dimensional network may cause the one or more types of hydrogel precursors to gel. As used herein, the term "hydrogel precursor" refers to any uncrosslinked hyaluronic acid polymer that may be used to form a hydrogel. hydrogel precursors are the amino-modified hyaluronic acid comprising a disulfide bond (HA-SS-NH₂).

The hydrogel precursor includes a chemically-modified polymer. The chemically-modified HA polymer may form a three-dimensional network in an aqueous medium to form a hydrogel. The chemically-modified HA polymer comprises a disulfide bond and terminal amine group.

The primary amine of the functionalized hyaluronic acid can be reacted with a chemical crosslinker. The hyaluronic acid polymer precursor (e.g., the uncrosslinked hyaluronic acid polymer) comprises a terminal amino group. The primary amine group enables the functionalized hyaluronic acid to be chemically crosslinked. In some embodiments, the chemical crosslinker is polyethylene glycol (PEG) comprising a succinimidyl functional group (e.g., -NHS), as shown in **FIG. 1B****.** In some embodiments, the crosslinker comprises a buffer (e.g., phosphate buffer). In some embodiments, the chemical crosslinker is a multi-arm PEG. In some embodiments, the chemical crosslinker is a 3-arm PEG, a 4-arm PEG, a 6-arm PEG, an 8-arm PEG, or any combination thereof. In some embodiments, the chemical crosslinker is an 8-arm PEG. As used herein, the term "multi-arm PEG" refers to a molecule having multiple linear PEG chains attached to a central core. For example, a 3-arm PEG refers to a molecule having 3 PEG chains coupled to its central core, a 4-arm PEG refers to a molecule having 4 PEG chains coupled to its central core, a 6-arm PEG refers to a molecule having 6 PEG chains coupled to its central core, a 8-arm PEG refers to a molecule having 8 PEG chains coupled to its central core.

In some embodiments, the crosslinker (e.g., PEG-NHS) is present at a concentration of about 1% w/v to about 70% w/v (e.g., about 1% to about 10% w/v, about 1% to about 15% w/v, about 1% to about 20% w/v, about 1% to about 25% w/v, about 1% to about 29% w/v, about 5% to about 10% w/v, about 5% to about 15% w/v, about 5% to about 20% w/v, about 5% to about 25% w/v, about 5% to about 30% w/v, about 10% to about 15% w/v, about 10% to about 20% w/v, about 10% to about 25% w/v, about 10% to about 30% w/v, about 1% to about 50% w/v, about 1% to about 60% w/v, about 1% to about 70% w/v, about 50% to about 60% w/v, about 50% to about 70% w/v, about 5% to about 50% w/v, about 10% to about 50% w/v, about 20% to about 50% w/v, about 30% to about 50% w/v, or about 40% to about 50% w/v) in a buffer (e.g., phosphate buffer). In some embodiments, the crosslinker (e.g., PEG-NHS) is present at a concentration of about 10% w/v to in a buffer (e.g., phosphate buffer). In some embodiments, the crosslinker (e.g., PEG-NHS) is present at a concentration of about 50% w/v.

In some embodiments, the chemical crosslinker is PEG having a molecular weight ranging from about 5 kilodalton (kDa) to about 200 kDa (e.g., about 5 kDa to about 10 kDa, about 5 kDa to about 15 kDa, about 5 kDa to about 20 kDa, about 5 kDa to about 25 kDa, about 5 kDa to about 30 kDa, about 5 kDa to about 35 kDa, about 5 kDa to about 40 kDa, about 5 kDa to about 50 kDa, about 10 kDa to about 15 kDa, about 10 kDa to about 20 kDa, about 10 kDa to about 25 kDa, about 10 kDa to about 30 kDa, about 10 kDa to about 35 kDa, about 10 kDa to about 40 kDa, about 10 kDa to about 50 kDa, about 15 kDa to about 20 kDa, about 15 kDa to about 25 kDa, about 15 kDa to about 30 kDa, about 15k Da to about 35 kDa, about 15 kDa to about 40 kDa, about 15 kDa to about 50 kDa, about 20 kDa to about 25 kDa, about 20 kDa to about 30 kDa, about 20 kDa to about 35 kDa, about 20 kDa to about 40 kDa, about 20 kDa to about 50 kDa, about 25 kDa to about 30 kDa, about 25 kDa to about 35 kDa, about 25 kDa to about 40 kDa, about 25 kDa to about 50 kDa, about 30 kDa to about 35 kDa, about 30 kDa to about 40 kDa, about 30 kDa to about 50 kDa, about 35 kDa to about 40 kDa, about 35 kDa to about 50 kDa, or about 40 kDa to about 50 kDa, about 5 kDa to about 100 kDa, about 5 kDa to about 150 kDa, about 5 kDa to about 200 kDa, about 50 kDa to about 100 kDa, about 50 kDa to about 150 kDa, about 50 kDa to about 200 kDa, about 100 kDa to about 150 kDa, about 100 kDa to about 200 kDa, or about 150 kDa to about 200 kDa). In some embodiments, the chemical crosslinker is PEG having a molecular weight of about 10 kDa. In some embodiments, the chemical crosslinker is PEG having a molecular weight of about 40 kDa.

In some embodiments, the chemical crosslinker comprises one or more PEG polymers having different molecular weights (e.g., any of the above-described molecular weights of PEG). For example, in some embodiments, the chemical crosslinker is a combination of a first PEG having a first molecular weight and a second PEG having a second molecular weight that is different than the first molecular weight. In some embodiments, the first PEG has a molecular weight of about 10 kDa, and the second PEG has a molecular weight of about 40 kDa. In some embodiments, the chemical crosslinker comprises a first PEG polymer and a second PEG polymer at a ratio of about 0:100 wt% to about 100:0 wt% (e.g., about 0:100 wt% to about 10:90 wt%, about 0:100 wt% to about 20:80 wt%, about 0:100 wt% to about 30:70 wt%, about 0:100 wt% to about 40:60 wt%, about 0:100 wt% to about 50:50 wt%, about 0:100 wt% to about 60:40 wt%, about 0:100 wt% to about 70:30 wt%, about 0:100 wt% to about 80:20 wt%, about 0:100 wt% to about 90:10 wt%, about 10:90 wt% to about 100:0 wt%, about 20:80 wt% to about 100:0 wt%, about 30:70 wt% to about 100:0 wt%, about 40:60 wt% to about 100:0 wt%, about 50:50 wt% to about 100:0 wt%, about 60:40 wt% to about 100:0 wt%, about 70:30 wt% to about 100:0 wt%, about 80:20 wt% to about 100:0 wt%, or about 90:10 wt% to about 100:0 wt%). In some embodiments, the chemical crosslinker comprises a first PEG polymer and a second PEG polymer at a ratio of about 70:30 wt%. In some embodiments, the chemical crosslinker comprises a first PEG polymer having a molecular weight of about 40 kDa and a second PEG polymer having a molecular weight of about 10 kDa at a ratio of about 70:30 wt%, respectively.

While the above-discussed hyaluronic acid polymer has been described and illustrated with respect to certain material formulations and methods of preparation, in some embodiments, a hyaluronic acid polymer that is otherwise substantially similar in formulation and function to the above-discussed hyaluronic acid polymer may include one or more materials formulations that are different from the ones discussed above or may be prepared using methods that are modified as compared to the methods described above. For example, while the hyaluronic acid polymer has been described and illustrated as including a terminal amino group, in some embodiments, a hyaluronic acid polymer that is otherwise substantially similar in formulation and function to the above-described hyaluronic acid polymer may alternatively include a thiol group instead of the amino group, as illustrated in **FIG. 4****.**

While above-discussed hyaluronic acid polymer has been described and illustrated as being crosslinked with a PEG crosslinker comprising a succinimidyl functional group, in some embodiments, a PEG crosslinker that is otherwise substantially similar in formulation and function to the above-discussed PEG crosslinker may include an ortho-pyridyl disulfide (OPSS) functional group instead of a succinimidyl functional group, as illustrated in **FIG. 4****.** For example, in some embodiments, the PEG crosslinker including the OPSS functional group crosslinks a chemically-modified HA polymer that is functionalized with a thiol group. In some embodiments, a PEG crosslinker that is otherwise substantially similar in formulation and function to the above-discussed PEG crosslinker may include an maleimide functional group instead of a succinimidyl functional group. For example, in some embodiments, the PEG crosslinker including the maleimide functional group crosslinks a chemically-modified HA polymer that is functionalized with a thiol group. In some embodiments, a PEG crosslinker that is otherwise substantially similar in formulation and function to the above-discussed PEG crosslinker may include an acrylate functional group instead of a succinimidyl functional group. For example, in some embodiments, the PEG crosslinker including the acrylate functional group crosslinks a chemically-modified HA polymer that is functionalized with a thiol group and/or an amine group. In some embodiments, the chemical crosslinker comprises one or more amine groups and a disulfide bond (e.g., NHS-SS-NHS).

### Physical Properties of MN Array Compositions

The physical properties of the microneedle array compositions of the disclosure include, but are not limited to, swelling ratio, mechanical strength, degradation rate, and drug release profile can be finely tuned by modulating the concentration, type, and/or molecular weight of the chemical crosslinker and/or the reducing agent. For example, in some embodiments, the molecular weight of the crosslinker can have a significant impact on the swelling ratio and mechanical strength of the amino-functionalized HA polymer. In some embodiments, the degradation rate is dependent on the concentration of the reducing agent that the amino-functionalized HA polymer is exposed to.

In some embodiments, the molecular weight of the crosslinker (e.g., PEG) is directly proportional to the swelling ratio of the hydrogel microneedles when measured after an extended period of time (e.g., 24 hours). In some embodiments, the molecular weight of the crosslinker does not have a significant impact on the swelling ratio of the hydrogel microneedles when measured after a short period of time (e.g., at about 2 hours at most). For example, as shown in **FIG. 7A****,** crosslinking the HA polymer with a crosslinker (e.g., PEG) having a high molecular weight (e.g., 40 kDa) can lead to greater swelling ratio (e.g., about 1800%) after about 24 hours. In some embodiments, the microneedles have a swelling ratio ranging from about 600% to about 1300% (e.g., about 600% to about 700%, about 600% to about 800%, about 600% to about 900%, about 600% to about 1000%, about 600% to about 1100%, about 600% to about 1200%, about 600% to about 1250%, about 900% to about 1000%, about 900% to about 1100%, about 900% to about 1200%, or about 900% to about 1300%) after contacting the functionalized HA hydrogels with an aqueous environment for at least about 15 minutes to about 2 hours. In some embodiments, the microneedles have a swelling ratio ranging from about 1300% to about 1800% (e.g., about 1300% to about 1400%, about 1300% to about 1500%, about 1300% to about 1600%, about 1300% to about 1700%, about 1300% to about 1750%, about 1400% to about 1500%, about 1400% to about 1600%, about 1400% to about 1700%, about 1400% to about 1800%, about 1500% to about 1600%, about 1500% to about 1700%, about 1500% to about 1800%, about 1600% to about 1700%, about 1600% to about 1800%, or about 1700% to about 1800%) after contacting the functionalized HA hydrogels with an aqueous environment for at least about 2 hours to about 48 hours (e.g., about 2 hours to about 12 hours, about 2 hours to 24 hours, about 2 hours to 36 hours, about 2 hours to 48 hours, about 12 hours to 24 hours, about 12 hours to 36 hours, about 12 hours to 48 hours, about 24 hours to 36 hours, about 24 hours to 48 hours, or about 36 hours to 48 hours).

In some embodiments, the molecular weight of the crosslinker (e.g., PEG) is inversely proportional to the mechanical strength of the microneedles. For example, crosslinking the HA polymer with a crosslinker (e.g., PEG) having a low molecular weight (e.g., 10 kDa) can lead to greater mechanical strength that results in more efficient skin perforation and/or penetration as compared to a crosslinker (e.g., PEG) having a higher molecular weight (e.g., 40 kDa). In some embodiments, crosslinking the HA polymer with a combination of crosslinkers (e.g., PEG) having a low molecular weight (e.g., 10 kDa) and a higher molecular weight (e.g., 40 kDa) at specific ratios (e.g., 70% (wt%) 40 kDa and 30% (wt%) 10 kDa) can lead to greater mechanical strength that results in more efficient skin perforation and/or penetration as compared to a single crosslinker (e.g., PEG) having a higher molecular weight (e.g., 40 kDa).

In some embodiments, the degradation of the crosslinked HA polymer is controlled by the time and concentration of the reducing agent that the hydrogel is exposed to. In some embodiments, the crosslinked HA polymer hydrogels have on-demand degradation (e.g., the degradation can be rapidly dissolved or degraded within 30 seconds or less). In some embodiments, the concentration of the reducing agent is directly proportional to the degradation time. As shown in **FIGs. 7D** and **7E****,** the degradation of the hydrogels is almost instantaneous (e.g., within about 5 minutes or less) when exposed to about 100 mM of a reducing agent (e.g., TCEP). In some embodiments, the crosslinked HA polymer hydrogel is degraded within about 15 seconds (s) to about 10 minutes (min.) (e.g., about 15 s to about 1 min., about 15 s to about 2 min., about 15 s to about 3 min., about 15 s to about 4 min., about 15 s to about 5 min., about 15 s to about 6 min., about 15 s to about 7 min., about 15 s to about 8 min., about 15 s to about 9 min., about 15 s to about 10 min., about 30 s to about 1 min., about 30 s to about 2 min., about 30 s to about 3 min., about 30 s to about 4 min., about 30 s to about 5 min., about 30 s to about 6 min., about 30 s to about 7 min., about 30 s to about 8 min., about 30 s to about 9 min., about 30 s to about 10 min., about 1 min. to about 2 min., about 1 min. to about 3 min., about 1 min. to about 4 min., about 1 min. to about 5 min., or about 1 min. to about 10 min.) when exposed to about 75 mM to about 150 mM (e.g., about 75 mM to about 100 mM, about 80 mM to about 100 mM, about 90 mM to about 100 mM, about 100 mM to about 110 mM, about 100 mM to about 115 mM, about 100 mM to about 120 mM, about 100 mM to about 130 mM, about 100 mM to about 140 mM, or about 100 mM to about 150 mM) of a reducing agent (e.g., TCEP).

In some embodiments, the crosslinked HA polymer hydrogel is degraded within about 30 seconds (s) to about 20 minutes (min.) (e.g., about 30 s to about 1 min., about 30 s to about 2 min., about 30 s to about 3 min., about 30 s to about 4 min., about 30 s to about 5 min., about 30 s to about 6 min., about 30 s to about 7 min., about 30 s to about 8 min., about 30 s to about 9 min., about 30 s to about 10 min., about 1 min. to about 2 min., about 1 min. to about 3 min., about 1 min. to about 4 min., about 1 min. to about 5 min., about 1 min. to about 10 min., about 30 s to about 11 min., about 30 s to about 12 min., about 30 s to about 13 min., about 30 s to about 14 min., about 30 s to about 15 min., about 30 s to about 16 min., about 30 s to about 17 min., about 30 s to about 18 min., about 30 s to about 19 min., about 30 s to about 19 min., about 1 min. to about 15 min., about 1 min. to about 20 min., about 5 min. to about 10 min., about 5 min. to about 15 min., about 5 min. to about 20 min., about 10 min. to about 15 min., about 10 min. to about 20 min., or about 15 min. to about 20 min.).

In some embodiments, the crosslinked HA polymer hydrogel is degraded when exposed to about 75 mM to about 150 mM (e.g., about 75 mM to about 100 mM, about 80 mM to about 100 mM, about 90 mM to about 100 mM, about 100 mM to about 110 mM, about 100 mM to about 115 mM, about 100 mM to about 120 mM, about 100 mM to about 130 mM, about 100 mM to about 140 mM, or about 100 mM to about 150 mM) of a reducing agent (e.g., TCEP).

In some embodiments, the crosslinked HA polymer hydrogel is degraded within about 10 min. to about 30 min. (e.g., about 10 min. to about 12 min., about 10 min. to about 15 min., about 10 min. to about 20 min., about 10 min. to about 25 min., about 10 min. to about 28 min., about 15 min. to about 20 min., about 15 min. to about 25 min., about 15 min. to about 30 min., about 20 min. to about 25 min., about 20 min. to about 30 min., or about 25 min. to about 30 min.) when exposed to about 5 mM to about 15 mM (e.g., about 5 mM to about 7.5 mM, about 5 mM to about 10 mM, about 5 mM to about 12.5 mM, about 5 mM to about 15 mM, about 10 mM to about 12.5 mM, or about 10 mM to about 15 mM) of a reducing agent (e.g., TCEP).

In some embodiments, the crosslinked HA polymer hydrogel is degraded within about 10 min. to about 30 min. (e.g., about 10 min. to about 12 min., about 10 min. to about 15 min., about 10 min. to about 20 min., about 10 min. to about 25 min., about 10 min. to about 28 min., about 15 min. to about 20 min., about 15 min. to about 25 min., about 15 min. to about 30 min., about 20 min. to about 25 min., about 20 min. to about 30 min., or about 25 min. to about 30 min.).

In some embodiments, the crosslinked HA polymer hydrogel is degraded when exposed to about 5 mM to about 15 mM (e.g., about 5 mM to about 7.5 mM, about 5 mM to about 10 mM, about 5 mM to about 12.5 mM, about 5 mM to about 15 mM, about 10 mM to about 12.5 mM, or about 10 mM to about 15 mM) of a reducing agent (e.g., TCEP).

In some embodiments, the crosslinked HA polymer hydrogel is degraded within about 50 hours (h) to about 150 h (e.g., about 50 h to about 75 h, about 50 h to about 100 h, about 50 h to about 125 h, about 50 h to about 150 h, about 75 h to about 100 h, about 75 h to about 125 h, about 75 h to about 150 h, about 100 h to about 125 h, about 100 h to about 150 h, about 125 h to about 150 h) when exposed to about 0.5 mM to about 2 mM (e.g., about 0.5 mM to about 1 mM, about 0.5 mM to about 1.25 mM, about 0.5 mM to about 1.5 mM, about 0.5 mM to about 1.75 mM, about 0.5 mM to about 1.9 mM, about 1 mM to about 1.25 mM, about 1 mM to about 1.5 mM, about 1 mM to about 1.75 mM, about 1 mM to about 2 mM, about 1.25 mM to about 1.5 mM, about 1.25 mM to about 1.75 mM, about 1.25 mM to about 2 mM, about 1.5 mM to about 1.75 mM, about 1.5 mM to about 2 mM, or about 1.75 mM to about 2 mM) of a reducing agent (e.g., TCEP).

In some embodiments, the crosslinked HA polymer hydrogel is degraded within about 50 hours (h) to about 150 h (e.g., about 50 h to about 75 h, about 50 h to about 100 h, about 50 h to about 125 h, about 50 h to about 150 h, about 75 h to about 100 h, about 75 h to about 125 h, about 75 h to about 150 h, about 100 h to about 125 h, about 100 h to about 150 h, about 125 h to about 150 h).

In some embodiments, the crosslinked HA polymer hydrogel is degraded when exposed to 0.5 mM to about 2 mM (e.g., about 0.5 mM to about 1 mM, about 0.5 mM to about 1.25 mM, about 0.5 mM to about 1.5 mM, about 0.5 mM to about 1.75 mM, about 0.5 mM to about 1.9 mM, about 1 mM to about 1.25 mM, about 1 mM to about 1.5 mM, about 1 mM to about 1.75 mM, about 1 mM to about 2 mM, about 1.25 mM to about 1.5 mM, about 1.25 mM to about 1.75 mM, about 1.25 mM to about 2 mM, about 1.5 mM to about 1.75 mM, about 1.5 mM to about 2 mM, or about 1.75 mM to about 2 mM) of a reducing agent (e.g., TCEP).

### Therapeutic Agents

In some embodiments, the HA hydrogel microneedles described herein include one or more therapeutic agents (e.g., as a drug delivery payload). In some embodiments, the one or more therapeutic agents are encapsulated within the polymeric, three-dimensional structure of each microneedle or other chemically modified HA polymeric structure or composition (e.g., a hydrogel disk or a hydrogel spray composition). In some embodiments, the therapeutic agents are encapsulated in, carried by, dispersed within, or otherwise loaded in or on the hydrogel microneedles or other chemically modified HA polymeric structure or composition (e.g., a hydrogel disk or a hydrogel spray composition). In some embodiments, one or more therapeutic agents are conjugated to a surface of the hydrogel microneedles or other chemically modified HA polymeric structure or composition (e.g., a hydrogel disk or a hydrogel spray composition). In some embodiments, a first therapeutic agent is encapsulated in, carried by, or otherwise loaded in or on a nanoparticle, a liposome, a micelle, a microparticle, an exosome, or the like. In some embodiments, a first therapeutic agent is encapsulated in, carried by, or otherwise loaded in or on a nanoparticle, a liposome, a micelle, a microparticle, an exosome, or the like, and a second therapeutic agent is outside the nanoparticle, liposome, micelle, microparticle, exosome, or the like within the same microneedle or other chemically modified HA polymeric structure or composition (e.g., a hydrogel disk or a hydrogel spray composition). In some embodiments, the first and second therapeutic agents are the same. In some embodiments, the first and second therapeutic agents are the different. In some embodiments, the therapeutic agents are dispersed, embedded, suspended, and/or mixed within the plurality of microneedles or other chemically modified HA polymeric structures.

In some embodiments, the plurality of microneedles or other chemically modified HA polymeric structure or composition (e.g., a hydrogel disk or a hydrogel spray composition) encapsulate a therapeutic agent at an encapsulation efficiency ranging from about 20% to about 95% (e.g., about 20% to about 25%, about 20% to about 30%, about 20% to about 40%, about 20% to about 50%, about 20% to about 55%, about 20% to about 60%, about 20% to about 65%, about 20% to about 70%, about 20% to about 75%, about 20% to about 80%, about 20% to about 85%, about 20% to about 90%, about 20% to about 95%, about 25% to about 30%, about 50% to about 55%, about 50% to about 60%, about 55% to about 60%, about 65% to about 70%, about 65% to about 75%, about 65% to about 80%, about 65% to about 85%, or about 65% to about 95%).

In some embodiments, the therapeutic agent is a hydrophobic therapeutic agent, a hydrophilic therapeutic agent, an amphiphilic therapeutic agent, or any combination thereof. In some embodiments, the therapeutic agent comprises an immunosuppressor. In some embodiments, the therapeutic agent comprises an immunoregulator. In some embodiments, the therapeutic agent comprises one or more of a chemokine, a chemotherapeutic, a nucleic acid, a protein (e.g., an antibody), a macromolecule, a nanoparticle, an exosome, a cytokine, a chemical-based drug (e.g., an anti-inflammatory therapeutic agent, a psychotropic drug, and/or anti-hypertensive drug), a growth factor, a vaccine, an immunosuppressor, an immunoregulator, or any combination thereof. In some embodiments, the therapeutic agent is a combination of two or more of any of the therapeutic agents listed herein. For example, in some embodiments, the HA hydrogel microneedles described herein comprise an anti-hypertensive drug (e.g., minoxidil) and one or more chemokines. In some embodiments, the HA hydrogel microneedles described herein comprise an anti-hypertensive drug (e.g., minoxidil) and one or more cytokines. In some embodiments, the HA hydrogel microneedles described herein comprise one or more chemokines and/or cytokines and one or more antibodies. In some embodiments, the HA hydrogel microneedles described herein comprise one or more chemokines and/or cytokines and one or more immunosuppressants (e.g., rapamycin).

In some embodiments, the therapeutic agent comprises C-C motif chemokine 22 (CCL22) and/or interleukin-2 (IL-2). In some embodiments, the therapeutic agent is a chemokine comprising chemokine (C-C motif) ligand 1 (CCL1), (C-C motif) ligand 2 (CCL2), (C-C motif) ligand 3 (CCL3), (C-C motif) ligand 4 (CCL4), (C-C motif) ligand 5 (CCL5), (C-C motif) ligand 6 (CCL6), (C-C motif) ligand 7 (CCL7), (C-C motif) ligand 8 (CCL8), (C-C motif) ligand 9 (CCL9), (C-C motif) ligand 10 (CCL10), (C-C motif) ligand 11 (CCL11), (C-C motif) ligand 12 (CCL12), (C-C motif) ligand 13 (CCL13), (C-C motif) ligand 14 (CCL14), (C-C motif) ligand 15 (CCL15), (C-C motif) ligand 16 (CCL16), (C-C motif) ligand 17 (CCL17), (C-C motif) ligand 18 (CCL18), (C-C motif) ligand 19 (CCL19), (C-C motif) ligand 20 (CCL20), (C-C motif) ligand 21 (CCL21), (C-C motif) ligand 22 (CCL22), (C-C motif) ligand 23 (CCL23), (C-C motif) ligand 24 (CCL24), (C-C motif) ligand 25 (CCL25), (C-C motif) ligand 26 (CCL26), (C-C motif) ligand 27 (CCL27), (C-C motif) ligand 28 (CCL28), chemokine (C-X-C motif) ligand 1 (CXCL1), chemokine (C-X-C motif) ligand 2 (CXCL2), chemokine (C-X-C motif) ligand 3 (CXCL3), chemokine (C-X-C motif) ligand 4 (CXCL4), chemokine (C-X-C motif) ligand 5 (CXCL5), chemokine (C-X-C motif) ligand 6 (CXCL6), chemokine (C-X-C motif) ligand 7 (CXCL7), chemokine (C-X-C motif) ligand 8 (CXCL8), chemokine (C-X-C motif) ligand 9 (CXCL9), chemokine (C-X-C motif) ligand 10 (CXCL10), chemokine (C-X-C motif) ligand 11 (CXCL11), chemokine (C-X-C motif) ligand 12 (CXCL12), chemokine (C-X-C motif) ligand 13 (CXCL13), chemokine (C-X-C motif) ligand 14 (CXCL14), chemokine (C-X-C motif) ligand 15 (CXCL15), chemokine (C-X-C motif) ligand 16 (CXCL16), chemokine (C-X-C motif) ligand 17 (CXCL17), chemokine (C motif) ligand 1 (XCL1), chemokine (C motif) ligand 2 (XCL2), chemokine (C-X3-C motif) ligand 1 (CX3CL1), or any combination thereof. In some embodiments, the therapeutic agents included in the HA hydrogel microneedles comprise one or more of any the chemokines described herein in combination with one or more of a cytokine, an antibody, an immunosuppressant (e.g., rapamycin), an anti-hypertensive (e.g., minoxidil), or any combinations thereof.

In some embodiments, the therapeutic agent is a chemokine receptor. In some embodiments, the chemokine receptor comprises CC chemokine receptor 1 (CCR1), CC chemokine receptor 2 (CCR2), CC chemokine receptor 3 (CCR3), CC chemokine receptor 4 (CCR4), CC chemokine receptor 5 (CCR5), CC chemokine receptor 6 (CCR6), CC chemokine receptor 7 (CCR7), CC chemokine receptor 8 (CCR8), CC chemokine receptor 9 (CCR9), CC chemokine receptor 10 (CCR10), CC chemokine receptor 11 (CCR11 ), CXC chemokine receptor 1 (CXCR1), CXC chemokine receptor 2 (CXCR2), CXC chemokine receptor 3 (CXCR3), CXC chemokine receptor 4 (CXCR4), CXC chemokine receptor 5 (CXCR5), CXC chemokine receptor 6 (CXCR6), or any combination thereof. In some embodiments, the therapeutic agents included in the HA hydrogel microneedles comprise one or more of any the chemokine receptors described herein in combination with one or more of a cytokine, an antibody, an immunosuppressant (e.g., rapamycin), an anti-hypertensive (e.g., minoxidil), or any combinations thereof.

In some embodiments, the therapeutic agent is a cytokine. In some embodiments, the immunosuppressor and/or the immunoregulator is a cytokine. In some embodiments, the cytokine comprises an interleukin (e.g., IL-2, IL-4, IL-7, IL-9, IL-13 and IL-15, IL-3, IL-6, IL-11, IL-13, IL-17 A-F, IL-21, IL-22, IL-23, IL10, IL-35), transforming growth factor-beta (TGF-β), interferon-gamma (IFN-γ), tumor necrosis factor-alpha (TNF-α), or any combination thereof. In some embodiments, the therapeutic agents included in the HA hydrogel microneedles comprise one or more of any the cytokines described herein in combination with one or more of a chemokine, an antibody, an immunosuppressant (e.g., rapamycin), an anti-hypertensive (e.g., minoxidil), or any combinations thereof.

In some embodiments, the therapeutic agent is an anti-neoplastic agent or a chemotherapeutic. As used herein, an "anti-neoplastic agent" is any substance used to treat cancer. This includes treatments to a primary tumor (e.g., to inhibit tumor growth), treatments to reduce invasiveness of a primary tumor, and treatments to inhibit metastasis. Anti-neoplastic agents include chemotherapeutic agents (e.g., small organic molecules, generally with a molecular weight less than 1 kDa), therapeutic proteins (e.g., antibodies, restriction enzyme, tumor suppressor protein) and therapeutic nucleic acids (e.g., DNAs (e.g., triplex-forming olignucleeotide, cDNA encoding an anti-neoplastic agent (e.g., an antibody)) and RNAs (e.g., siRNA, antisense RNA, ribozyme)). Anti-neoplastic agents often, though not exclusively, block one or more aspects of cell growth and/or proliferation (e.g., they can be cytostatic and/or cytotoxic). As used herein the term "chemotherapeutic agent" or "chemotherapeutic" (or "chemotherapy", in the case of treatment with a chemotherapeutic agent) is meant to encompass any non-proteinaceous (i.e., non-peptidic), non-nucleic acid chemical compound useful in the treatment of cancer.

In some embodiments, the chemotherapeutic comprises methotrexate, cisplatin, doxorubicin, docetaxel, erlotinib, paclitaxel, paraclitaxel, 5-fluorouracil and gemcitabine. Examples of chemotherapeutic agents include alkylating agents such as thiotepa and cyclophosphamide (CYTOXAN^{™}); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylolomelamine; acetogenins (e.g., bullatacin and bullatacinone); camptothecin (including synthetic analogues topotecan and irinotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, foremustine, lomustine, nimustine, ranimustine; antibiotics such as the enediyne antibiotics (e.g. calicheamicin, especially calicheamicin gamma1I and calicheamicin phiI1); dynemicin, including dynemicin A; bisphosphonates, such as clodronate; an esperamicin; as well as neocarzinostatin chromophore and related chromoprotein enediyne antibiotic chromomophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubincin (AdramycinTM) (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as demopterin, methotrexate, pteropterin, trimetrexate; purine analogs such as fludarabine, 6-mercaptopurine, thiamiprine, thioguanine; pyrimidine analogues such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenishers such as folinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK^{™}; razoxane; rhizoxin; sizofiran; spirogermanium; tenuazonic acid; triaziquone; 2,2',2"-trichlorotriethylamine; trichothecenes (e.g., T-2 toxin, verracurin A, roridin A and anguidine); urethane; vindesine; dacarbazine; mannomustine; mitobronitol; mitolactol; pipobroman; gacytosine; cytosine arabinoside ("Ara-C"); cyclophosphamide; thiopeta; taxoids, e.g. paclitaxel (TAXOL^{®}, Bristol Meyers Squibb Oncology, Princeton, N.J.) and docetaxel (TAXOTERE^{®}, Rhone-Poulenc Rorer, Antony, France); chlorambucil; gemcitabine (GemzarTM); 6-thioguanine; mercaptopurine; methotrexate; platinum analogs such as cisplatin (DDP) and carboplatin; vinblastine; platinum; etoposide (VP-16); ifosfamide; mitroxantrone; vancristine; vinorelbine (Navelbine^{™}); novantrone; teniposide; edatrexate; daunomycin; aminopterin; xeoloda; ibandronate; CPT-11; topoisomerase inhibitor RFS 2000; difluromethylornithine (DMFO); retinoids such as retinoic acid; capecitabine; and pharmaceutically acceptable salts, acids or derivatives of any of the above.

Also included in the definition of "chemotherapeutic agent" are anti-hormonal agents that act to regulate or inhibit hormone action on tumors such as anti-estrogens and selective estrogen receptor modulators (SERMs), including, for example, tamoxifen (including Nolvadex^{™}), raloxifene, droloxifene, 4-hydroxytamoxifen, trioxifene, keoxifene, LY117018, onapristone, and toremifene (Fareston^{™}); inhibitors of the enzyme aromatase, which regulates estrogen production in the adrenal glands, such as, for example, 4(5)-imidazoles, aminoglutethimide, megestrol acetate (Megace^{™}), exemestane, formestane, fadrozole, vorozole (Rivisor^{™}), letrozole (Femara^{™}), and anastrozole (Arimidex^{™}); and antiandrogens such as flutamide, nilutamide, bicalutamide, leuprolide, and goserelin; and pharmaceutically acceptable salts, acids or derivatives of any of the above.

In some embodiments, an anti-neoplastic agent is a tyrosine kinase inhibitor. For example, ZD1839 (Iressa^{™} of AstraZeneca K.K.) shows a competitive effect for ATP in ATP binding site of EGFR (epidermal growth factor receptor) tyrosine kinase, and inhibits tyrosine kinase activity by inhibiting autophosphorylation of tyrosine kinase. Another inhibitor of EGFR tyrosine kinase activity is erlotinib (N-(3-ethynylphenyl)-6,7-bis(2-methoxyethoxy)quinazolin-4-amine). Imatinib mesylate (GLEEVEC^{™}, formerly STI-571) can inhibit the tyrosine kinase activity of both BCR-Abl and c-kit. Sorafenib (Nexavar^{™}) is a small molecular inhibitor of Raf kinase, PDGF (platelet-derived growth factor) VEGF receptor 2 & 3 kinases and c-Kit.

Anti-neoplastic agents can also include therapeutic antibodies, including those raised against tumor antigens or antigens associated with myelodysplasia. Therapeutic antibodies include antibody fragments, as discussed above, monoclonal antibodies, chimeric antibodies and humanized antibodies. Exemplary therapeutic antibodies include the following. IMC-C225 or cetuximab (Erbitux^{™}), is an EGFR-targeted monoclonal antibody that recognizes the receptor portion of EGFR on the cell surface and inhibits the autophosphorylation of EGFR; thereby inhibiting its tyrosine kinase activity. Herceptin (trastuzumab) is a monoclonal antibody directed against the Her2/Neu protein (which is homologous to EGFR and whose overexpression is associated with more aggressive disease and poorer prognosis, particularly in breast cancers). Rituximab (RITUXAN^{™}) is an antibody raised against the CD20 protein on lymphoma cells and which selectively depletes normal and malignant CD20+ pre-B and mature B cells. Alemtuzumab (CAMPATH^{™}) is a monoclonal antibody that specifically targets the CD52 antigen found on B and T lymphocytes; it is used for the treatment of chronic lymphocytic leukemia (CLL) and lymphoma. Gemtuzumab zogamicin (MYLOTARG^{™}) is an antibody conjugate that combines a specific antibody directed against CD33 with a chemotherapeutic drug (zogamicin), and is indicated for the treatment of relapsed adult acute myelocytic leukemia.

In some embodiments, the therapeutic agent comprises a nucleic acid. In some embodiments, the therapeutic agent can include a naturally occurring, modified, or synthetic nucleic acid such as, but not limited to, deoxyribonucleic acid (DNA), ribonucleic acid (RNA), viral vectors, chromosomes, aptamers, nucleosomes, or any combination thereof. Non-limiting examples of nucleic acids include DNA such as genomic DNA, methylated DNA, specific methylated DNA sequences, fragmented DNA, mitochondrial DNA, and RNA/DNA hybrids. Non-limiting examples of nucleic acids also include RNA such as various types of coding and non-coding RNA. Examples of the different types of RNA include messenger RNA (mRNA), ribosomal RNA (rRNA), transfer RNA (tRNA), microRNA (miRNA), viral RNA, CRISPR RNA (crRNA), trans-activating CRISPR RNA (tracrRNA), single guide RNA (sgRNA), and crRNA/tracrRNA hybrid. The RNA can be small (e.g., less than 200 nucleic acid bases in length) or large (e.g., RNA greater than 200 nucleic acid bases in length). Small RNAs mainly include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA), and small rDNA-derived RNA (srRNA). The RNA can be double-stranded RNA or single-stranded RNA. The RNA can be circular RNA. The RNA can be a bacterial rRNA (e.g., 16s rRNA or 23s rRNA). Further exemplary nucleic acids include, but are not limited to, recombinant nucleic acids, recombinant DNA, cDNA, genomic DNA, dsDNA, RNA, siRNA, mRNA, saRNA, miRNA, lncRNA, tRNA, and shRNA. In some embodiments, the nucleic acid is homologous to a nucleic acid in a cell. In some embodiments, the nucleic acid is heterologous to a nucleic acid in a cell. In some embodiments, the nucleic acid is in the form of a plasmid. In some embodiments, the nucleic acid is a therapeutic nucleic acid. In some embodiments, the nucleic acid encodes a therapeutic polypeptide.

In some embodiments, the therapeutic agent comprises a protein. In some embodiments, the therapeutic agent comprises a polypeptide, or peptide, including, but not limited to, a structural protein, an enzyme, a cytokine (such as an interferon and/or an interleukin), a polyclonal or monoclonal antibody, or an effective part thereof, such as an Fv fragment, which antibody or part thereof, can be natural, synthetic or humanized, a peptide hormone, a receptor, or a signaling molecule. In some embodiments, the therapeutic agent comprises insulin. In some embodiments, the therapeutic agent comprises a hormone (e.g. a thyroid hormone such as levothyroxine or synthetic triiodothyronine). In some embodiments, the protein comprises an antibody (e.g., a single variable domain on a heavy chain (VHH) antibody, a nanobody^{®}). In some embodiments, the antibody is anti-CD3 monoclonal antibody. In some embodiments, the antibody comprises an anti-programmed death-1 (PD-1) monoclonal antibody, an anti-programmed death ligand-1 (PD-1) monoclonal antibody, an anti-vascular endothelial growth factor receptor (VEGFR) monoclonal antibody, an anti-cytotoxic T-lymphocyte-associated protein 4 (CTLA4) monoclonal antibody, or any combination thereof. In some embodiments, the antibody comprises an anti-CD3 monoclonal antibody, an anti-IL-6 monoclonal antibody, an anti-CD28 monoclonal antibody, an anti-CD52 monoclonal antibody, or any combination thereof. In some embodiments, the therapeutic agents included in the HA hydrogel microneedles comprise one or more of any the antibodies described herein in combination with one or more of a chemokine, a cytokine, an immunosuppressant (e.g., rapamycin), an anti-hypertensive (e.g., minoxidil), or any combinations thereof.

In some embodiments, the therapeutic agent comprises a macromolecule. In some embodiments, macromolecule comprises glucose. In some embodiments, the therapeutic agent includes, but is not limited to, at least one of a protein, a polypeptide, a peptide, a nucleic acid, a virus, a virus-like particle, an amino acid, an amino acid analogue, a modified amino acid, a modified amino acid analogue, a steroid, a proteoglycan, a lipid and a carbohydrate or a combination thereof (e.g., chromosomal material comprising both protein and DNA components or a pair or set of effectors, wherein one or more convert another to active form, for example catalytically).

In some embodiments, the therapeutic agent comprises a nanoparticle. Non-limiting examples of nanomaterials or nanoparticles that can be delivered using the methods and compositions of the disclosure include quantum dots, plasmonic nanoparticles, metallic nanoparticles, polymeric nanoparticles, liposomes, lipid nanoparticles, exosomes, or any combination thereof. In some embodiments, the nanomaterial or nanoparticle has a size (e.g. a diameter) of less than about 300 nm. In some embodiments, the nanomaterial or nanoparticle can have a diameter of between about 2 nm to about 200 nm (e.g., between about 10 nm to about 30 nm, between about 5 nm to about 25 nm, between about 10 nm to about 25 nm, between about 15 nm to about 25 nm, between about 20 nm and about 25 nm, between about 25 nm to about 50 nm, between about 50 nm and about 200 nm, between about 70 nm and about 200 nm, between about 80 nm and about 200 nm, between about 100 nm and about 200 nm, between about 140 nm to about 200 nm, and between about 150 nm to about 200 nm).

In some embodiments, the nanomaterial or nanoparticle can be spherical or ellipsoidal, or can have an amorphous shape. In some embodiments, the nanomaterial or nanoparticle can be magnetic (e.g., include a core of a magnetic material). In some embodiments, the magnetic material or particle can contain a diamagnetic, paramagnetic, superparamagnetic, or ferromagnetic material that is responsive to a magnetic field.

In some embodiments, the nanomaterial or nanoparticle can contain, in part, a core and/or a shell of containing a polymer (e.g., poly(lactic-co-glycolic acid)). Skilled practitioners will appreciate that any number of art known materials can be used to prepare nanoparticles, including, but are not limited to, gums (e.g., Acacia, Guar), chitosan, gelatin, sodium alginate, and albumin. Additional polymers that can be used to generate the nanomaterial or nanoparticle to be dispersed in the cell solution are known in the art. For example, polymers that can be used to generate the nanomaterial or nanoparticle include, but are not limited to, cellulosics, poly(2-hydroxy ethyl methacrylate), poly(N-vinyl pyrrolidone), poly(methyl methacrylate), poly(vinyl alcohol), poly(acrylic acid), polyacrylamide, poly(ethylene-co-vinyl acetate), poly(ethylene glycol), poly(methacrylic acid), polylactides (PLA), polyglycolides (PGA), poly(lactide-co-glycolides) (PLGA), polyanhydrides, polyorthoesters, polycyanoacrylate and polycaprolactone.

In some embodiments, the chemical-based drug comprises an anti-inflammatory therapeutic agent. In some embodiments, the therapeutic agent is a corticosteroid. Exemplary chemical-based drug for inclusion in the compositions include, but are not limited to, an antibacterial agent, an anti-fungal agent, an anti-viral agent, an anti-acanthamoebal agent, an immunosuppressive agent, an anti-vascular endothelial growth factor (anti-VEGF) agent, a growth factor, or any combination thereof.

In some embodiments, the therapeutic agent is a psychotropic drug (e.g., an antidepressant or an anti-epileptic drug). Non-limiting examples of anti-depressants include a tricyclic antidepressant (e.g. secondary amine tricyclic antidepressant or a tertiary amine tricyclic antidepressant), a selective serotonin reuptake inhibitor, a serotonin and noradrenaline reuptake inhibitor, a reversible monoamine oxidase inhibitor and a monoamine oxidase inhibitor, nortriptyline, desipramine, amitriptyline, imipramine, fluoxetine, citalopram, paroxetine, fluvoxamine, escitalopram (lexapro), sertraline, venlafaxine, moclobemide, phenelzine, duloxetine, and tranylcypromine. Non-limiting examples of anti-epileptic drugs includes carbamazepine, valproate, ethosuximide and phenyloin. In some embodiments, the psychotropic drug is arapiprazole, olanzapine, quetiapine, risperidone, or ziprasidone.

In some embodiments, the therapeutic agent is an anti-inflammatory therapeutic agent. Non-limiting examples of suitable anti-inflammatory agents include a steroidal anti-inflammatory drug (e.g., prednisolone), a non-steroidal anti-inflammatory drug (e.g., bromfenac), an mTOR inhibitor (e.g., rapamycin), a calcineurin inhibitor, a synthetic or natural anti-inflammatory protein, methylprednisolone, prednisolone, hydrocortisone, fludrocortisone, prednisone, celecoxib, ketorolac, piroxicam, diclorofenac, ibuprofen, and ketoprofen, rapamycin, cyclosporin, and tacrolimus/FK-506. In some embodiments, the therapeutic agent is an anti-hypertensive drug. Non-limiting examples of anti-hypertensive drugs include a vasodilator (e.g., minoxidil), an angiotensin-converting enzyme (ACE) inhibitors (e.g., captopril, benazepril, enalapril, enalaprilat, fosinopril, lisinopril, quinapril, ramipril, and trandolapril or others with similar molecular mechanisms), a calcium channel blocker (e.g., nifedipine, verapamil, nicardipine, diltiazem, isradipine, amlodipine, nimodipine, felodipine, nisoldipine, bepridil or others with similar molecular mechanisms), a beta-blocker (e.g., atenolol, metoprolol, propranolol, timolol, nadolol, acebutolol, pindolol, sotalol, labetalol, oxprenolol or others with similar molecular mechanisms), methyldopa, hydralazine hydrochloride, labetalol, adenosine, nifedipine, and magnesium sulfate. In some embodiments, the therapeutic agents included in the HA hydrogel microneedles comprise any of the anti-hypertensive drugs listed herein (e.g., minoxidil) and one or more chemokines and/or cytokines. In some embodiments, the therapeutic agents included in the HA hydrogel microneedles comprise one or more of any the anti-hypertensive drugs described herein in combination with one or more of a chemokine, a cytokine, an antibody, an immunosuppressant (e.g., rapamycin), or any combinations thereof.

In some embodiments, the therapeutic agent is a growth factor. In some embodiments, the growth factor includes, but is not limited to, epithelial growth factor, fibroblast growth factor, nerve growth factor, hepatocyte growth factor, or any combination thereof. Further non-limiting examples of suitable growth factors include transforming growth factors (TGFs) (e.g., beta transforming growth factors such as, TGF-β1, TGF-β2, TGF-β3), fibroblast growth factors (FGFs), platelet derived growth factors (PDGFs), epidermal growth factors (EGFs), connective tissue activated peptides (CTAPs), osteogenic factors, bone morphogenetic proteins (e.g., BMP-1, BMP-2, BMP-3, BMP-4, BMP-5, BMP-6, BMP-7, BMP-8, BMP-9); heparin-binding growth factors (e.g., fibroblast growth factor (FGF), epidermal growth factor (EGF), insulin-like growth factor (IGF)), Inhibins (e.g., Inhibin A, Inhibin B), growth differentiating factors (for example, GDF-1), and Activins (e.g.,, Activin A, Activin B, Activin AB), and biologically active analogs, fragments, and derivatives of such growth factors.

In some embodiments, the therapeutic agent is a vaccine. In some embodiments, the therapeutic agent is an mRNA vaccine. In some embodiments, the therapeutic agent is a vaccine comprise an RNA that encodes highly immunogenic antigens capable of eliciting potent neutralizing antibodies responses against coronavirus antigens, such as Severe Acute Respiratory Syndrome (SARS)-CoV-2 coronavirus antigens. In some embodiments, the therapeutic agent is a "booster" vaccine. As used herein, when referring to a prophylactic composition, such as a vaccine, the term "booster" refers to an extra administration of the prophylactic (vaccine) composition. A booster (or booster vaccine) may be given after an earlier administration of the prophylactic composition.

In some embodiments, the hydrogel composition further includes a pharmaceutically acceptable carrier. As used herein, the expression "pharmaceutically acceptable carrier" refers to a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluent, excipient, solvent, or encapsulating material, or a combination thereof. Each component of the carrier must be "pharmaceutically acceptable" in that it must be compatible with the other ingredients of the formulation and is compatible with administration to a subject, for example a human. It must also be suitable for use in contact with any tissues or organs with which it may come in contact, meaning that it must not carry a risk of toxicity, irritation, allergic response, immunogenicity, or any other complication that excessively outweighs its therapeutic benefits. Examples of pharmaceutically acceptable carriers include, but are not limited to, a solvent or dispersing medium containing, for example, water, pH buffered solutions (e.g., phosphate buffered saline (PBS), HEPES, TES, MOPS, etc.), isotonic saline, Ringer's solution, polyol (for example, glycerol, propylene glycol, liquid polyethylene glycol, and the like), alginic acid, ethyl alcohol, and suitable mixtures thereof. In some embodiments, the pharmaceutically acceptable carrier can be a pH buffered solution (e.g. PBS).

In some embodiments, the pharmaceutically acceptable carrier is a topical carrier. In some embodiments, the composition is formulated for topical use. In some embodiments, the composition is topically administered to a tissue (e.g., a skin tissue) of a patient. In some embodiments, the composition can be applied to a tissue (e.g., a skin tissue) for topical, targeted delivery of a therapeutic agent.

### Methods of Microneedle Fabrication

The present disclosure features methods of preparing a microneedle array using the above-described hyaluronic acid polymer comprising a disulfide bond and a terminal amine group. A variety of methods for manufacturing microneedles are available and any suitable method for manufacturing microneedles or microneedle arrays are contemplated for use with the compositions and methods disclosed herein. In some embodiments, microneedles are manufactured using any suitable method, including, but not limited to molding (e.g., self-molding, micro-molding, micro-embossing, microinjection, roll-to-roll processing, and the like).

In some embodiments, the methods include casting the hyaluronic acid polymer solution into a microneedle mold. In some embodiments, the microneedle mold comprises an array of negative microneedle projections (e.g., an array of 11 x 11 microneedles). In some embodiments, the negative microneedle projections have a height within any of the height ranges described elsewhere herein (e.g., of about 600 µm) and circular base having a radius within any of the radius or width ranges described elsewhere herein (e.g., a radius of about 150 µm). The microneedle mold can be composed of any suitable material (e.g., polydimethylsiloxane (PDMS)).

Next, in some embodiments, the methods include centrifuging the microneedle mold containing the hyaluronic acid polymer solution. In some embodiments, the centrifugation step forces the HA polymer solution throughout the mold and helps to evenly distribute the HA polymer solution. In some embodiments, the microneedle mold containing the hyaluronic acid polymer solution is centrifuged at a speed of about 3000 revolutions per minute (RPM) to about 5000 (e.g., about 3000 RPM to about 4200 RPM, about 3500 RPM to about 4200 RPM, about 4000 RPM to about 4200 RPM, about 4200 RPM to about 4500 RPM, or about 4200 RPM to about 5000 RPM). In some embodiments, the microneedle mold containing the hyaluronic acid polymer solution is centrifuged at about 4200 RPM. In some embodiments, the microneedle mold containing the hyaluronic acid polymer solution is centrifuged for about 1 min. to about 10 mins (e.g., about 1 min. to about 5 min., about 2 min. to about 5 min., about 3 min. to about 5 min., about 4 min. to about 5 min., about 5 min. to about 6 min., about 5 min. to about 7 min., about 5 min. to about 8 min., about 5 min. to about 9 min., or about 5 min. to about 10 min.). In some embodiments, the microneedle mold containing the hyaluronic acid polymer solution is centrifuged for about 5 minutes.

Next, in some embodiments, the methods include freeze-drying the microneedle mold containing the hyaluronic acid polymer solution. In some embodiments, the microneedle mold containing the hyaluronic acid polymer solution is freeze-dried for about 12 hours or more. Next, in some embodiments, the methods include casting a crosslinker (e.g., PEG-NHS) solution into the microneedle mold containing the freeze-dried hyaluronic acid polymer to crosslink the hyaluronic acid polymer and form a hydrogel. In some embodiments, the centrifugation step forces the crosslinker solution throughout the mold and helps to evenly distribute the crosslinker solution throughout the freeze-dried polymer composition. In some embodiments, the methods described herein enable a gradual gelation of the HA hydrogel and ensure a successful polymerization of the hydrogel matrix (e.g., from the tip to the base of each microneedle) that has a homogenous composition.

In some embodiments, the microneedle mold containing the crosslinker solution and freeze-dried polymer composition is centrifuged at a speed of about 3000 revolutions per minute (RPM) to about 5000 (e.g., about 3000 RPM to about 4200 RPM, about 3500 RPM to about 4200 RPM, about 4000 RPM to about 4200 RPM, about 4200 RPM to about 4500 RPM, or about 4200 RPM to about 5000 RPM). In some embodiments, the microneedle mold containing the crosslinker solution and freeze-dried polymer composition is centrifuged at about 4200 RPM. In some embodiments, the microneedle mold containing the crosslinker solution and freeze-dried polymer composition is centrifuged for about 1 min. to about 10 mins (e.g., about 1 min. to about 5 min., about 2 min. to about 5 min., about 3 min. to about 5 min., about 4 min. to about 5 min., about 5 min. to about 6 min., about 5 min. to about 7 min., about 5 min. to about 8 min., about 5 min. to about 9 min., or about 5 min. to about 10 min.). In some embodiments, the microneedle mold containing the crosslinker solution and freeze-dried polymer composition is centrifuged for about 5 minutes. In some embodiments, excess polymer and/or crosslinker solution is carefully removed after the centrifugation step. In some embodiments, the methods include freeze-drying the microneedle mold containing the crosslinked hydrogel. In some embodiments, the microneedle mold containing the crosslinked hydrogel is freeze-dried for about 12 hours or more.

Next, in some embodiments, a solution containing one or more therapeutic agents (e.g., chemokines) is deposited in the microneedle mold containing the freeze-dried, crosslinked hydrogel. In some embodiments, the microneedle mold containing the added therapeutic agent is spun for about 1 second (s) to about 60 s (e.g., about 1 s to about 15 s, about 1 s to about 30 s, about 1 s to about 45 s, about 1 s to about 60 s, about 15 s to about 30 s, about 15 s to about 45 s, about 15 s to about 60 s). In some embodiments, the microneedle mold containing the added therapeutic agent is subjected to a vacuum pressure for about 1 min. to about 5 min. (e.g., about 1 min. to about 2 min., about 1 min. to about 3 min., about 1 min. to about 4 min., or about 1 min. to about 5 min.). In some embodiments, the microneedle mold containing the added therapeutic agent is subjected to a vacuum pressure instead of being spun. In some embodiments, the microneedle mold containing the added therapeutic agent is subjected to a vacuum pressure in addition to being spun (e.g., before or after microneedle mold is spun).

In some embodiments, an end-capping agent is added to the therapeutic agent. In some embodiments, the end-capping agent is added to the chemically-modified HA hydrogels prior to adding the therapeutic agent. In some embodiments, the end-capping agent and the therapeutic agent are simultaneously added to the chemically-modified HA hydrogels. In some embodiments, the end-capping agent is an agent comprising amino-end groups that can preferentially interact with the non-reacted N-hydroxysuccinimide (NHS)-terminal groups of the crosslinker. In some embodiments, the end-capping agent prevents the therapeutic agent from binding to the chemically-modified HA hydrogels. In some embodiments, the end-capping agent is glycine. In some embodiments, the end-capping agent is an agent comprising a primary amine group. In some embodiments, the end-capping agent is hydroxylamine. In some embodiments, the addition of an end-capping agent can prevent any amine groups present in the therapeutic agent (e.g., a chemokine or cytokine) from interacting and/or binding to the NHS-terminal groups of the crosslinker. In some embodiments, when the therapeutic agent comprises an amine group, the addition of an end-capping agent can result in at least 80% therapeutic agent delivery from the chemically-modified HA hydrogels as compared to chemically-modified HA hydrogels that do not include the end-capping agent (e.g., glycine or hydroxylamine). In some embodiments, the end-capping agent (e.g., glycine or hydroxylamine) is added at a concentration of about 1 µg/ml to about 50 µg/ml (e.g., about 1 µg/ml to about 10 µg/ml, about 5 µg/ml to about 10 µg/ml, about 10 µg/ml to about 15 µg/ml, about 10 µg/ml to about 20 µg/ml, about 10 µg/ml to about 25 µg/ml, about 10 µg/ml to about 30 µg/ml, about 10 µg/ml to about 35 µg/ml, about 10 µg/ml to about 40 µg/ml, about 10 µg/ml to about 45 µg/ml, about 10 µg/ml to about 50 µg/ml). In some embodiments, the end-capping agent (e.g., glycine or hydroxylamine) is added at a concentration of about 10 µg/ml. In some embodiments, the end-capping agent is added at a concentration of about 1:10 (chemically-modified HA:end-capping agent) mol/mol. In some embodiments, the end-capping agent is added at a concentration of about 1:10 (end-capping agent:chemically-modified HA) mol/mol.

Next, in some embodiments, the methods include adding a backing layer to the microneedle mold containing the freeze-dried hyaluronic acid hydrogel and the therapeutic agent solution. In some embodiments, the backing layer is added immediately after spinning the microneedle mold containing the added therapeutic agent. In some embodiments, the backing layer is the substrate of the microneedle array. In some embodiments, the backing layer is a polymeric layer. In some embodiments, the backing layer comprises a biodegradable polymer. In some embodiments, the biodegradable polymer is a poly(D,L-lactide-co-glycolide) (PLGA) polymer. In some embodiments, any suitable biodegradable polymer can be used to prepare the backing layer. Non-limiting examples of biodegradable polymers include, but are not limited to, polymers of hydroxy acids such as lactic acid and glycolic acid polylactide, polyglycolide, polylactide-co-glycolide, and copolymers with PEG, polyanhydrides, poly(ortho)esters, polyurethanes, poly(butyric acid), poly(valeric acid), and poly(lactide-co-caprolactone). In some embodiments, the backing layer is formed by adding a polymeric solution to the mold. In some embodiments, the polymeric solution is added dropwise until covering the entire surface of the mold.

In some embodiments, the methods further include drying the microneedle mold containing the hyaluronic acid hydrogel and the backing layer. In some embodiments, the microneedle mold is dried for at least about 12 hours. In some embodiments, the microneedle mold is dried at about room temperature (e.g., about 20 °C to about 25 °C). Lastly, In some embodiments, the methods include removing the microneedle mold from the microneedle array. In some embodiments, the microneedle mold is peeled off. In some embodiments, the microneedle mold is stored in a low-humidity environment (e.g., about 1% relative humidity (rh) to about 20% rh) or a no-humidity environment (e.g., 0% rh).

### Methods of Delivery and/or Sampling

The present disclosure features methods of transdermally delivering a therapeutic agent to a subject in need thereof using the chemically-modified hyaluronic acid microneedle array compositions described herein (e.g., the microneedle arrays composed of the above-described hyaluronic acid polymer comprising a disulfide bond and a terminal amine group).

These methods of delivering a therapeutic agent are not according to the invention as claimed.

In some embodiments, the methods include contacting any of the microneedle array compositions described herein with a skin surface of the subject. Next, in some embodiments, the methods further include applying pressure on the microneedle array such that the penetrating tip of each microneedle of the plurality of microneedles penetrates the skin surface. In some embodiments, the tip portion of the microneedles perforates and/or penetrates the stratum corneum. In some embodiments, the microneedles penetrate the epidermis and/or the dermis. In some embodiments, the microneedles create a pathway or pore within a skin tissue (e.g., within the stratum corneum, epidermis, and/or dermis) and release the therapeutic agent. In some embodiments, once released, the therapeutic agent can directly go into the systemic circulation without facing the barrier of the stratum corneum. In some embodiments, once released, the therapeutic agent can directly go into the lymphatic circulation without facing the barrier of the stratum corneum. In some embodiments, once released, the therapeutic agent can remain within a localized area of the tissue near the microneedle injection site. In some embodiments, the therapeutic agent is internalized by cells at, near, and/or neighboring the microneedle injection site.

In some embodiments, the methods include maintaining the microneedle array in place for about 5 minutes to about 1 week (e.g., about 5 min. to about 10 min., about 5 min. to about 15 min., about 5 min. to about 20 min., about 5 min. to about 30 min., about 5 min. to about 1 hour (h), about 5 min. to about 2 h, about 5 min. to about 3 h, about 5 min. to about 6 h, about 5 min. to about 12 h, about 5 min. to about 18 h, about 5 min. to about 24 h, about 5 min. to about 36 h, about 5 min. to about 48 h, about 5 min. to about 72 h, about 5 min. to 4 days, about 5 min. to 5 days, about 5 min. to 6 days, about 5 min. to 7 days, about 10 min. to about 15 min., about 10 min. to about 20 min., about 10 min. to about 30 min., about 10 min. to about 1 hour (h), about 10 min. to about 2 h, about 1 h to about 2 h, about 1 h to about 3 h, about 1 h to about 6 h, about 1 h to about 12 h, about 1 h to about 24 h, about 1 day to about 2 days, about 1 day to about 2 days, about 1 day to about 3 days, about 1 day to about 4 days, about 1 day to about 5 days, about 1 day to about 6 days, about 1 day to about 7 days, or more) after the penetrating tip of each microneedle of the plurality of microneedles penetrates the skin surface.

The present disclosure features methods include of sampling an interstitial fluid of a subject in need thereof. In some embodiments, the methods include simultaneous delivery of a therapeutic agent and sampling of an interstitial fluid. In some embodiments, the methods include delivery of a therapeutic agent without sampling of the interstitial fluid. In some embodiments, the methods sampling of the interstitial fluid without simultaneous delivery of a therapeutic agent. In some embodiments, the methods include steps similar as those described above for delivery of a therapeutic agent. For example, in some embodiments, the methods include contacting the microneedle array of the disclosure with a skin surface of the subject. Next, in some embodiments, the methods further includes applying pressure on the microneedle array such that the penetrating tip of each microneedle of the plurality of microneedles penetrates the skin surface, thereby absorbing the interstitial fluid.

In some embodiments, the hydrogel microneedles can absorb the skin interstitial fluid once they penetrate through the stratum corneum, epidermis, and/or dermis. In some embodiments, the methods further include removing the microneedle array from the skin surface after the interstitial fluid is absorbed. In some embodiments, the methods include removing the microneedle array from the skin surface after a period of time ranging from about 5 min. to about 24 hours (e.g., about 5 min. to about 10 min., about 5 min. to about 15 min., about 5 min. to about 30 min., about 5 min. to about 1 h, about 5 min. to about 2 h, about 5 min. to about 3 h, about 5 min. to about 6 h, about 5 min. to about 12 h, about 5 min. to about 18 h, about 5 min. to about 24 h, about 30 min. to about 1 h, about 1 h to about 2 h, about 1 h to about 4 h, about 1 h to about 6 h, about 1 h to about 12 h, about 1 h to about 24 h, about 6 h to about 12 h, about 6 h to about 18 h, about 6 h to about 24 h, about 12 h to about 18 h, or about 12 h to about 24 h). Next, in some embodiments, the methods include degrading the plurality of microneedles after removing the microneedle array from the skin surface. In some embodiments, the plurality of microneedles is degraded by contacting the plurality of microneedles with a reducing agent, thereby extracting the interstitial fluid from the plurality of microneedles. In some embodiments, contacting the plurality of microneedles with a reducing agent does not cause cell death and does not negatively affect (e.g., degrade, contaminate, or otherwise interfere with the stability of) one or more components of the interstitial fluid (e.g., a cell, biomarker, antibody, or any combination thereof). In some embodiments, contacting the plurality of microneedles with a reducing agent does not impedes the phenotyping of one or more components of the interstitial fluid (e.g., a cell, biomarker, antibody, or any combination thereof) (e.g., there is no cleavage/degradation of cell receptors). In some embodiments, the degrading of the plurality of microneedles is facilitated by the cleavage of the disulfide bond present in the degradable HA polymer. In other words, in some embodiments, the disulfide bond is configured to be cleaved upon exposure to the reducing agent. In some embodiments, the reducing agent is TCEP. In some embodiments, the reducing agent is glutathione, dithiothreitol, or beta-mercaptoethanol. In some embodiments, the plurality of microneedles is contacted with the reducing agent at any of the reducing agent concentrations disclosed elsewhere herein.

In some embodiments, the interstitial fluid comprises a biomarker and/or a cell. In some embodiments, the biomarker is a cytokine or a chemokine (e.g., any of the cytokines and/or chemokines disclosed elsewhere herein). In some embodiments, the biomarker is glucose. In some embodiments, the biomarker is caffeine. In some embodiments, the biomarker is an orally- and/or systemically-administered drug (e.g., nonsteroidal anti-inflammatory drug such as ibuprofen, naproxen, or the like). In some embodiments, the hydrogel microneedles can be coupled with a wearable device for in situ detection of biomarkers using electrochemical and optical techniques. For example, in some embodiments, the microneedle array can extract a skin interstitial fluid comprising glucose, and the methods disclosed herein include determining a level of glucose in the ISF that can be indicative of a blood glucose level of a patient. In some embodiments, the wearable device can be a continuous glucose monitor that is operatively connected with the microneedle array.

Non-limiting examples of one or more biomarkers that can be detected in situ using the microneedle array described herein include, but are not limited to, bilirubin, carnosine, cortisol, creatine, creatinine, homocysteine, uric acid, vitamin A, vitamin B12, lumazine, pantothenic acid (vitamin B5), lumichrome, pyridoxamine (vitamin B6 form), pyridoxal (vitamin B6 form), 4-pyridoxic acid, ascorbic acid (vitamin C), ergocalciferol (vitamin D2), 7-dehydrocholesterol (vitamin D3), hypoxanthine, nicotinamide adenine dinucleotide (NAD), uridine, xanthine, myristic acid (c14:0), palmitoleic acid (c16:1), stearic acid (c18:0), arachidic acid (c20:0), cholic acid, glycocholic acid, urocanic acid, 4-Guanidinobutanoic acid, succinylhomoserine, tocopherol, N6-(delta2-isopentenyl)-adenine, nebularine, cytidine monophosphate (CMP), cytidine, inosine, 3-methyladenine, nicotinamide ribotide, N-methyltryptamine, sphingosine, 20-COOH-leukotriene B4, stachyose, gulonolactone, fructose 6-phosphate, rhamnose, oxalic acid, phosphoenolpyruvic acid, diethanolamine, cyclohexane-1,2-diol, triethanolamine, methyl jasmonate, or any combinations thereof. In some embodiments, the interstitial fluid biomarker that can be sampled with the methods and microneedle arrays described herein comprises a sugar, salt, fatty acid, amino acid, coenzyme, enzyme, hormone, neurotransmitter, or any combination thereof.

In some embodiments, one or more cells found in the interstitial fluid can be sampled with the methods and microneedle arrays described herein comprises. In some embodiments, the one or more cells that can be sampled include, but are not limited to, T-cell, B-cell, a natural killer (NK) cell, a neutrophil, a macrophage, a monocyte, a dendritic cell, a memory T cell, a regulator T cell, a myeloid-derived suppressor cell (MDSC), or any combination thereof. In some embodiments, the microneedles of the disclosure can release a therapeutic agent that attracts a specific type of cell and further recruit and extract that specific type of cell. In some embodiments, the cell recruited and extracted by the microneedle array compositions of the disclosure is an immune cell. In some embodiments, the immune cell is a T-cell, B-cell, a natural killer (NK) cell, a neutrophil, a macrophage, a monocyte, a dendritic cell, a memory T cell, a regulator T cell, a myeloid-derived suppressor cell (MDSC),or any combination thereof. In some embodiments, the cell is a T cell (e.g., a regulatory T cell). In some embodiments, the therapeutic agent used to recruit the cell is a cytokine, a chemokine, an antibody, or any combination thereof (e.g., any of the cytokines, chemokines, and/or antibodies disclosed elsewhere herein). In some embodiments, the microneedles of the disclosure can release a therapeutic agent that repels and/or depletes a specific type of cell within a target tissue site. For example, in some embodiments, the microneedles of the disclosure can release an anti-CD3 monoclonal antibody for T cell (e.g., CD3+-T cell) depletion therapy.

The present disclosure features methods of delivering a therapeutic agent to a subject in need thereof using the chemically-modified hyaluronic acid hydrogel compositions described herein (e.g., hydrogels composed of the above-described hyaluronic acid polymer comprising a disulfide bond and a terminal amine group).

In some embodiments, the methods include contacting the subject with a chemically crosslinked hydrogel comprising a hyaluronic acid polymer comprising a disulfide bond. In some embodiments, as described elsewhere herein, the hydrogel can be configured to be degraded upon exposure to a reducing agent. In some embodiments, the methods include using a chemically crosslinked hydrogel that is crosslinked with a crosslinker comprising PEG further comprising a succinimidyl functional group, as described elsewhere herein. In some embodiments, the hydrogel is an injectable hydrogel. For example, In some embodiments, the hydrogel is a viscous fluid that can be made to pass through needles as small as 27 gauge (G). In some embodiments, the hydrogel composition can have a thixotropic nature. For example, in some embodiments, the hydrogel will transform to a solution when subjected to sufficient shear force, rendering the hydrogel (and any therapeutic agents contained therein) injectable.

In some embodiments, the hydrogel is a pre-formed hydrogel depot. In some embodiments, the hydrogel depots (e.g., hydrogel implants) can release one or more therapeutic agents over extended periods of time (e.g., at least over 24 hours) in a controlled fashion to achieve an effective concentration without reaching toxic levels. In some embodiments, the hydrogel can be formulated to readily allow diffusion of the payload without requiring degradation of the hydrogel (e.g., bioerosion) for a controlled release of the therapeutic agent. In some embodiments, the hydrogel can be formulated to readily allow diffusion of the payload based on the degradation of the hydrogel (e.g., bioerosion) for a controlled release of the therapeutic agent. For example, in some embodiments, bioerosion of the hydrogel can be conducted once implanted by enzymes such as hyaluronidase. In some embodiments, the hydrogel can include a matrix structure that provides for diffusion of the therapeutic agent without depending on bioerosion, a process of making the hydrogel that allows for dispersion of the agent in the hydrogel, and providing for the agent to be encapsulated (e.g., within nanoparticles or microparticles) while being suspended in the hydrogel. In some embodiments, the therapeutic agent does not have to be encapsulated in particles, or otherwise combined with materials that need bioerosion to release them. In some embodiments, the hydrogels can be made to last longer than the therapeutic agents they deliver so that delivery is controlled and the release of a final burst of the therapeutic agent is kept within limits that avoid potentially toxic effects. In some embodiments, the hydrogel is a sprayable hydrogel. In some embodiments, the hydrogel is an aerosol. In some embodiments, the hydrogel is a sprayable hydrogel wound dressing.

### Indications

The present disclosure features methods of treating a disease in a subject in need thereof using the chemically-modified hyaluronic acid compositions described herein (e.g., the microneedle arrays composed of the above-described hyaluronic acid polymer comprising a disulfide bond and a terminal amine group). Methods of treatment are not according to the invention as claimed. The hydrogel compositions (e.g., hydrogel microneedle array compositions) of the disclosure may be used for the prevention and/or treatment of a wide variety of diseases; particularly cancers and skin disorders. Both benign and malignant tumors, as well as metastases of a primary tumor, can be treated.

As used herein, "prevention" includes prophylaxis, delay of onset of symptoms or blocking onset of symptoms altogether. As used herein, "treatment," refers to inhibition of progression of a neoplastic disease, malignant disorder, or skin disorder, stasis of symptoms, prolongation of survival, partial or full amelioration of symptoms, and partial or full eradication of a neoplastic condition, disease, or disorder or full eradication of a skin condition, disease, or disorder.

Thus, treatment includes partial or total alleviation of symptoms, or reduction of signs or symptoms of illness, and specifically includes, without limitation, prolongation of survival. The expected progression-free survival times may be measured in months to years, depending on prognostic factors including the number of relapses, stage of disease, and other factors. Prolongation of survival includes without limitation an increase in survival time of at least 1 month, about at least 2 months, about at least 3 months, about at least 4 months, about at least 6 months, about at least 1 year, about at least 2 years, about at least 3 years, or more. Overall survival can also be measured in months to years. The patient's symptoms may remain static or may decrease.

Non-limiting indications that can be treated using the hydrogel compositions of the disclosure include those involving undesirable or uncontrolled cell proliferation. Such indications include benign tumors, various types of cancers such as primary tumors and tumor metastasis, restenosis (e.g. coronary, carotid, and cerebral lesions), hematological disorders, abnormal stimulation of endothelial cells (atherosclerosis), insults to body tissue due to surgery, abnormal wound healing, abnormal angiogenesis, diseases that produce fibrosis of tissue, liver fibrosis, kidney fibrosis, lung fibrosis, scleroderma, atherosclerosis, repetitive motion disorders, disorders of tissues that are not highly vascularized, and proliferative responses associated with organ transplants.

Generally, cells in a benign tumor retain some or all of their differentiated features and do not divide in a completely uncontrolled manner. A benign tumor is usually localized and nonmetastatic. Specific types of benign tumors that can be treated using the present disclosure include, but are not limited to, hemangiomas, hepatocellular adenoma, cavernous haemangioma, focal nodular hyperplasia, acoustic neuromas, neurofibroma, bile duct adenoma, bile duct cystanoma, fibroma, lipomas, leiomyomas, mesotheliomas, teratomas, myxomas, nodular regenerative hyperplasia, trachomas and pyogenic granulomas.

In a malignant tumor, cells become undifferentiated, do not respond to the body's growth control signals, and multiply in an uncontrolled manner. Malignant tumors are invasive and capable of spreading to distant sites (metastasizing). Malignant tumors are generally divided into two categories: primary and secondary. Primary tumors arise in a particular tissue and remain in that tissue (i.e., they remain in situ). A secondary tumor, or metastasis, is a tumor which originates in one region of the body and spreads to another region. Common routes for metastasis of a malignant cell are direct growth into adjacent structures, dissemination through the vascular or lymphatic systems, and tracking along tissue planes and body spaces (peritoneal fluid, cerebrospinal fluid, etc.).

Primary and metastatic tumors that can be treated by the methods disclosed herein include, but are not limited to, lung cancer (including, but not limited to, lung adenocarcinoma, squamous cell carcinoma, large cell carcinoma, bronchioloalveolar carcinoma, non-small-cell carcinoma, small cell carcinoma, mesothelioma); breast cancer (including, but not limited to, ductal carcinoma, lobular carcinoma, inflammatory breast cancer, clear cell carcinoma, mucinous carcinoma); colorectal cancer (including, but not limited to, colon cancer, rectal cancer); anal cancer; pancreatic cancer (including, but not limited to, pancreatic adenocarcinoma, islet cell carcinoma, neuroendocrine tumors); prostate cancer; ovarian carcinoma (including, but not limited to, ovarian epithelial carcinoma or surface epithelial-stromal tumour including serous tumour, endometrioid tumor and mucinous cystadenocarcinoma, sex-cord-stromal tumor); liver and bile duct carcinoma (including, but not limited to, hepatocelluar carcinoma, cholangiocarcinoma, hemangioma); esophageal carcinoma (including, but not limited to, esophageal adenocarcinoma and squamous cell carcinoma); non-Hodgkin's lymphoma; bladder carcinoma; carcinoma of the uterus (including, but not limited to, endometrial adenocarcinoma, uterine papillary serous carcinoma, uterine clear-cell carcinoma, uterine sarcomas and leiomyosarcomas, mixed mullerian tumors); glioma, glioblastoma, medullablastoma, and other tumors of the brain; kidney cancers (including, but not limited to, renal cell carcinoma, clear cell carcinoma, Wilm's tumor); cancer of the head and neck (including, but not limited to, squamous cell carcinomas); cancer of the stomach (including, but not limited to, stomach adenocarcinoma, gastrointestinal stromal tumor); multiple myeloma; testicular cancer; germ cell tumors; neuroendocrine tumors; cervical cancer; carcinoids of the gastrointestinal tract, breast, and other organs; and signet ring cell carcinoma.

Mesenchymal tumors include, but are not limited to, sarcomas, fibrosarcomas, haemangioma, angiomatosis, haemangiopericytoma, pseudoangiomatous stromal hyperplasia, myofibroblastoma, fibromatosis, inflammatory myofibroblastic tumor, lipoma, angiolipoma, granular cell tumor, neurofibroma, schwannoma, angiosarcoma, liposarcoma, rhabdomyosarcoma, osteosarcoma, leiomyoma, and leiomysarcoma.

Specific types of cancers or malignant tumors, either primary or secondary, that can be treated using compositions disclosed herein also include, but are not limited to, skin cancer, bone cancer, brain cancer, cancer of the larynx, gall bladder, pancreas, parathyroid, thyroid, adrenal, neural tissue, head and neck, bronchi, basal cell carcinoma, squamous cell carcinoma of both ulcerating and papillary type, metastatic skin carcinoma, osteosarcoma, Ewing's sarcoma, veticulum cell sarcoma, myeloma, giant cell tumor, small-cell lung tumor, islet cell tumor, primary brain tumor, acute and chronic lymphocytic and granulocytic tumors, hairy-cell tumor, adenoma, hyperplasia, medullary carcinoma, pheochromocytoma, mucosal neuromas, intestinal ganglioneuromas, hyperplastic corneal nerve tumor, marfanoid habitus tumor, seminoma, ovarian tumor, leiomyomater tumor, cervical dysplasia and in situ carcinoma, neuroblastoma, retinoblastoma, soft tissue sarcoma, malignant carcinoid, topical skin lesion, mycosis fungoides, rhabdomyosarcoma, Kaposi's sarcoma, osteogenic and other sarcomas, malignant hypercalcemia, renal cell tumor, polycythermia vera, adenocarcinoma, glioblastoma multiforme, lymphomas, malignant melanomas and epidermoid carcinomas.

Proliferative responses and/or immune responses associated with organ transplantation that, for example, contribute to transplant rejection and associated complications can also be treated using the methods and compositions of the present disclosure. Specifically, these proliferative responses can occur during transplantation of organs such as the heart, lung, liver, and kidney; as well as following bone marrow or other hematopoietic cell transplantations. Furthermore, immune responses associated with skin transplantation (e.g., allografts) can also be treated using the methods and compositions of the present disclosure.

Non-limiting skin indications that can be treated using the hydrogel compositions of the disclosure include, but are not limited to, topic dermatitis, contact dermatitis, drug-induced delayed type cutaneous allergic reactions, toxic epidermal necrolysis, cutaneous T-cell lymphoma, bullous pemphigoid, alopecia areata, alopecia totalis, alopecia universalis, androgenetic alopecia, vitiligo, acne rosacea, prurigo nodularis, scleroderma, herpes simplex viral skin infections, acne, rosacea, eczema, keloids, psoriasis, pruritus, scleroderma, post-inflammatory hyperpigmentation, melasma, skin cancers, burns, lupus erythematosus, or any combination thereof.

In some embodiments, the hydrogel microneedle array compositions of the disclosure may be utilized to treat and/or prevent a variety of infectious diseases. Non-limiting skin infectious diseases that can be treated using the hydrogel compositions of the disclosure include, but are not limited to, Severe Acute Respiratory Syndrome (SARS), Middle East Respiratory Syndrome (MERS), and COVID-19 (Coronavirus Disease 2019). In some embodiments, the hydrogel microneedle array compositions treat and/or prevent the variety of infectious diseases by delivering a vaccine (e.g., an RNA vaccine) comprising and/or encoding highly immunogenic antigens capable of eliciting potent neutralizing antibodies responses against coronavirus antigens, such as SARS-CoV-2 coronavirus antigens.

### EXAMPLES

Certain embodiments of the present disclosure are further described in the following examples, which do not limit the scope of any embodiments described in the claims.

### Example 1 - Synthesis of HA-Modified Polymer

HA polymer was selected since it is a biocompatible, non-immunogenic, and FDA-approved linear polysaccharide. The natural ability of HA to absorb a large volume of water makes it an ideal candidate for rapid interstitial fluid (ISF) extraction. In addition, HA can function as a natural ligand of the ubiquitous CD44 receptor, providing a binding motif for the cells present in ISF. HA polymer was chemically modified to allow for the formation of a digestible HA hydrogel capable of extracting both cellular and soluble biomarkers that are present in ISF upon patch retrieval. HA was modified with cysteamine dihydrochloride molecule, harboring both a primary amine group (for hydrogel formation) and a disulfide bond (for hydrogel degradation) **(****FIG. 1B****).** To form the HA-based hydrogel, HA primary amines were reacted with the 8-arm-PEG-NHS crosslinker containing a succinimidyl functional group **(****FIG. 1B****),** allowing for spontaneous hydrogel formation without the use of external triggers. The disulfide bond in the modified HA backbone allowed for on demand cleavage to release the migrated cells following ISF sampling upon the addition of a reducing agent, tris (2-carboxyethil) phosphine (TCEP-a water-soluble and non-toxic reducing agent widely used for biochemical applications). The addition of TCEP facilitated the collapse of the tri-dimensional structure of the hydrogel MNs and in turn, the release of the entrapped cells **(****FIG. 1C****).**

60 kDa-sodium hyaluronate (1% w/v in MES buffer) was activated with N-(3-(dimethylamino)propyl)carbodiimide (EDC) and N-hydroxysuccinimide (NHS) at a 1:4:2 molar ratio and reacted at room temperature for 30 minutes (**FIG. 2A**). The activated hyaluronic acid (HA) was then mixed with cysteamine dihydrochloride at1:10 molar ratio and reacted at room temperature for 12 hours (**FIG. 2B**). HA-SS-NH₂ was purified by dialysis against deionized water for 6 days at room temperature, freeze dried, and stored at -20°C protected from humidity until use. For structural analysis, modified HA-SS-NH₂ was dissolved in D₂O and analyzed by ¹H-NMR, recorded using a 400 MHz Varian NMR spectrometer (**FIGs. 3A-3B**).

### Example 2 - Fabrication Process of Microneedles (MNs)

MNs were produced using custom-made molds having an 11 x 11 array of negative MN projections, each one with a height of about 600 µm and a radius of about 150 µm. First, HA-SS-NH₂ polymer (10% w/v in phosphate buffer, pH=7.4) was casted on top of the molds and centrifuged at 4200 rpm for 5 minutes. Excess polymer was removed, and molds were freeze-dried. Then, 8-arm-PEG-NHS crosslinker (10% w/v in phosphate buffer, pH=7.4) was casted and forced by centrifugation through the mold under the same conditions. This approach, together with the gradual gelation of hydrogel, ensured a successful polymerization of the matrix from "tip-to-top" of the MNs and a homogenous composition. Excess polymer was carefully removed, and molds were freeze-dried. Next, an aqueous solution containing chemokines and glycine (10 µg mL-1) was deposited and briefly spun for 15 seconds. Immediately after, a polymeric backing layer of PLGA (Resomer^{®} RG 858 S, Sigma-Aldrich, USA) at 15% (w/v) dissolved in acetonitrile was added dropwise until covering the whole area of the mold. Finally, HA-based MNs were allowed to dry at room temperature for 12 hours, peeled off the molds carefully, and stored at room temperature preserved from humidity.

HA-based MNs can be fabricated using any commercially available or custom-made mold, varying in their length (from 100µm to 1500µm), shape (pyramidal, conical, etc.), needle density, and center-to-center needle spacing. Following the described method, similar HA-based microneedles can be fabricated by pairing an HA-derived polymer and a PEG-based crosslinker including a disulfide bond (**FIG. 4**). Resulting MNs can be dissolved using the same strategy by adding a reductive agent.

### Example 3 - Biophysical Characterization of the HA-Based MNs

Biophysical properties of HA-based MNs can be tuned using crosslinkers with different molecular weight. Here, the use of PEG derivatives ranging from 10 kilodalton (kDa) to 40 kDa was assessed. The PEG-40 kDa-derived-hydrogels dissolved after 48 hours of incubation while the volume of the other hydrogels remained unchanged for extended periods of time (**FIG. 5**). In some embodiments, PEG derivatives having molecular weights lower than 10 kDa and molecular weights higher than 40 kDa can be used as crosslinkers.

Referring to **FIG. 7A**, an average swelling ratio of about 800% in less than an hour was observed for all formulations. The molecular weight of the crosslinker did not affect the initial swelling phase, but it had a strong impact into the final swelling rates. The molecular weight of the crosslinker dictates the MNs mechanical strength of the MNs. The ex vivo studies revealed that MNs containing low molecular weight crosslinker (10 kDa-PEG alone or when mixed with the 40 kDa-PEG) could efficiently disrupt the stratum corneum as confirmed by the accumulation of blue tissue-marking dye inside the micro-conduits (**FIG. 7B**). MNs fabricated using the 40 kDa-PEG were not capable of piercing the skin effectively. Same results were confirmed in in vivo settings as effective skin penetration was observed (**FIG. 7C**).

Fine-tuning the composition of the MNs by using different crosslinkers enables the production of MNs that have the required mechanical properties for skin penetration, thereby avoiding the use of solid core/shell MNs and irreversible crosslinking strategies as others have done before. Forming the entire structures of the microneedles from the hydrogel provides with a higher volume for entrapment of ISF biomarkers, thereby permitting a better diagnostic sensitivity.

### Example 4 - On-Demand Digestion of HA-Based MNs

### Cytotoxicity studies

The ability to induce on-demand digestion of the MNs using a reducing agent (Tris (2-carboxyethil) phosphine, TCEP) was also characterized. TCEP was found to be highly biocompatible and did not affect cell viability (**FIG. 6**). THP-1 cells were incubated with different concentrations of digestion media (TCEP solution in supplemented media), ranging from 0.1 mM to 100 mM, for 10 minutes. Thereafter, digestion media was removed, and cells were stained using the LIVE/DEAD fixable Violet Dead Cell Stain Kit (ThermoFisher Scientific, USA) following the manufacturer's guidelines. Dead cells were analyzed by flow cytometry using a BD LSRFortessa^{™} flow cytometer.

Study of the digestion kinetics of the HA-derived hydrogels confirmed a linear correlation between TCEP concentration and the time needed for full hydrogel digestion (**FIG. 7D**). High concentrations of TCEP (>10 mM) ensured complete digestion in half an hour whereas hydrogel digestion was not accomplished, or was far too long, when TCEP concentration was lower than 1 mM. Considering these findings, the working concentration for further studies was 10 mM TCEP.

Study of the digestion kinetics of HA-derived MNs confirmed complete digestion of the MN array in less than 5 minutes (**FIG. 7E**) when using a 10 mM TCEP solution. The MNs high surface-to-volume ratio and low overall volume (about 3 µL) (compared to the hydrogel disks) facilitated the penetration of the reducing agent and accelerated their digestion. Here, TCEP was selected due to its excellent biocompatibility, yet alternative reducing agents could be used for the same purpose such as glutathione, dithiothreitol, or beta-mercaptoethanol.

### Example 5 - HA-Based MNs for Drug Delivery

The release kinetics and drug delivery of the MNs were initially studied by using fluorescently-labeled IL-2 as a model drug. Interestingly, no IL-2 was released (**FIG. 8A**), which was attributed to the interaction between the amine groups of IL-2 and unreacted, free NHS groups of the crosslinker, which impeded its release. To validate this hypothesis, the interaction with the cytokine was blocked by treating the hydrogel with an end-capping agent, the amino acid glycine, whose amino end groups would preferentially interact with the crosslinker's non-reacted NHS-terminal groups. Indeed, end-capping the hydrogel resulted in more than 80% of the cytokine being released making it the chosen strategy for further studies.

Significant differences in the release profile were not observed when comparing MNs crosslinked with PEG molecules differing in their molecular weight. On average, an 80% of the cytokine was delivered within the first 30 minutes with a maximum delivered load of about 85% after 48 hours.

Contrarily, it was demonstrated that functionalization of the HA backbone with different pendant groups such as amino or thiol groups influenced drug retention and thus, their release profile (**FIG. 8B**). If MNs derived from the amino-modified HA released the cytokine in a burst-like fashion in early timepoints, MNs modified with thiol groups showed a sustained release profile and only a maximum of 50% of the total cytokine load was released.

Next, the loading efficiency of IL-2 was studied by fluorescence quantification, revealing a linear correlation between the loaded concentration and the retrieved concentration after digestion of the MN patches (**FIG. 8C**) confirming the accuracy of the loading method and the potential to deliver biologically relevant dosages.

### Example 6 - HA-Based MNs for ISF Sampling

In parallel, the diagnostic capacity of the platform and its ability to extract ISF in the absence of chemotactic agents was studied. Sampling of soluble biomarkers using MNs was investigated using a mimetic skin model. Briefly, agarose gels mimicking the mechanical properties of the epidermis/ISF were covered with a stretched layer of parafilm that emulated the water-impermeable stratum corneum. Hydrogel-based MNs were applied to the skin model containing a model analyte, Rhodamine B (RhoB), which was recovered after digestion of the patches.

Differences in RhoB concentration were easily detected by gross observation as evidenced by the color change of the MN matrix after administration (**FIG. 9A**). Quantification of the analyte absorbance confirmed a linear correlation between the concentration of RhoB in the skin-mimetic hydrogel and the concentration of the retrieved ISF when sampled using the PEG-40 kDa: 10 kDa-derived MNs (**FIG. 9B**).

Extraction of the cellular component of ISF was assessed by incubating the arrays of MNs in monocyte-like cell suspensions followed by their digestion to collect and measure the infiltrated cellular fraction. Arrays of solid MNs were also incorporated to the analysis as controls to discern whether recovered cells were embedded within the hydrogel matrix or originated from unspecific interactions with the MN walls. Analysis of the MN suspension after digestion by flow cytometry confirmed that the presence of cells, supporting that cells can diffuse into the hydrogel MNs and remain inside the matrix. As expected, the number of cells recovered from the solid arrays was practically negligible. Results also pointed at a correlation between the swelling ability of the hydrogel and its permissiveness to cell infiltration (**FIG. 9C**). It was found that higher number of cells appeared to be infiltrated within MNs formulated with the 40 kDa-crosslinker, which possesses the highest swelling ability, compared to the other formulations.

Hydrogel functionalization with pendant groups had a deep impact on cell infiltration and retention. Following incubation of the hydrogels with immune cells (THP-1 cells) in vitro, it was confirmed by light microscopy that certain formulations enhance cell infiltration. Cell infiltration was hypothesized to be favored by the electrostatic interactions between the functional groups and the negatively charged cellular membrane (**FIG. 9D**).

### Example 7 - Use of MNs for Cancer Theranostics

Checkpoint inhibitor (CPI) immunotherapies have shown great potential in the treatment of various cancers. Unfortunately, numerous patients with unfavorable disease phenotypes do not respond to CPI therapies as the highly immunosuppressive tumor microenvironments limits their efficacy. Delivery of immunostimulatory molecules such as the CpG DNA ligand of TLR9 is known to activate immune signaling and improve patient responsiveness to CPIs. For this reason, it was hypothesized that transdermal delivery of a CpG ligand with MNs could be used as a non-invasive strategy to prevent tumor growth and more favorable outcomes for CPI therapies. Specifically, the microneedle-based platform was proposed to be used for local delivery of CpG-encapsulated polymeric nanoparticles while surveilling the shifts in the CD8/CD4 ratios and other cells biomarkers such as natural killer cells (NKs) as a response to the treatment (**FIG. 10**).

For this work, a subcutaneous colorectal cancer model (MC38) was used where mice were administered poly(beta-amino ester) (PBAE)-polyplexes encapsulating the CpG ligand via: (1) hydrogel MNs or (2) intratumorally as a control. Following application, MNs were retrieved, and the recovered ISF was analyzed by flow cytometry for immunophenotyping. It was confirmed that the hydrogel MNs could be administered in the tumor area. The hydrogel MNs efficiently disrupted the epidermis layer, as seen in **FIG. 11A****,** to deliver their therapeutic cargo. Following the administration of the treatment, the data confirmed a significant reduction of tumor growth (**FIG. 11B**) and increased survival for all the individuals when compared to the untreated group (**FIG. 11C**).

Digestion of the MN patch upon retrieval allowed for the study the immune cell populations present in the ISF surrounding the tumor. The preliminary data showed that CD4 and CD8 cell populations could be distinguished by flow cytometry analysis, confirming the potential of the MNs as a non-invasive tool for ISF sampling (results not shown).

In this work, HA-based MNs containing nanoparticles that remained bioactive after the fabrication process were developed. MNs allowed for multiple rounds of therapy administration in the proximity of the tumor without compromising the skin integrity. Mice treated with CpG-loaded MNs reported a significant reduction of their tumor volume and prolonged survival. Simultaneously, MNs allowed to monitor immune populations such as CD4⁺ and CD8⁺ cells using the same patch, as the high swelling of the platform maximized ISF extraction (**FIG. 12**).

### Example 8 - Use of MNs for Management of Skin Transplant Rejection

Management of skin allograft rejection remains a milestone to be achieved. Following transplantation, trauma patients, albeit their immunosuppressed state, still mount a robust immune rejection against allogeneic skin as it is the most immunogenic of all known allografts. This, in turn, inevitably leads to graft loss. Clinically-used immunosuppressive regimens have been successful in preventing acute rejection in allografts, yet failed to protect against chronic rejection due to their deleterious effect on the regulatory arm of the immune system. Therefore, inducing immune regulation after allograft transplantation without impeding other protective immune functions is crucial to improve long-term outcome and guarantee donor-specific tolerance.

A new MN-based platform for local delivery of immunomodulators to manage skin allograft rejection was developed. Using hyaluronic acid as the base polymer, an MN patch capable of delivering chemokines such as CCL22 and IL-2 transdermally was designed (**FIG. 13A**). MNs can effectively penetrate the skin transplant without causing major scarring or trauma (**FIG. 13B**) and rapidly release the immunomodulators due to their high swelling abilities. Results confirmed that MNs could be loaded with bioactive immunomodulators such as CCL22 and IL-2. First, regulatory T cell (Treg) migration assays were conducted to assess the percentage of migrated Tregs as a function of CCL22 concentration. Results confirmed that loaded MNs could mediate similar, if not greater, levels of Treg recruitment compared to soluble chemokines, and where a dose-dependent trend was observed (**FIG. 13C**). After exploring the ability of the MNs to rapidly release chemokines in vitro, the MN local immune modulatory function was studied in vivo using a fully mismatched major histocompatibility complex (MHC) skin transplant model, which is a highly immunogenic model of rejection. Using quantitative polymerase chain reaction (PCR), differential expression of CD3 as universal T cell marker (cell infiltrates inducing allograft rejection), FoxP3 as a transcription factor differentiating Tregs from conventional T cells (Tregs suppress rejection), and IL-6 as a major pro-inflammatory cytokine involved in promoting graft rejection, were studied and normalized to the expression of the house keeping gene GAPDH. Skin allografts treated with CCL22+100 ng IL-2 delivered via MNs showed significantly increased ratio of FoxP3 to CD3 compared to allografts treated with empty MNs (p= 0.03) (**FIG. 13D**). Although skin grafts treated with CCL22 and lower IL-2 dose of 10 ng showed a trend of higher FoxP3 to CD3 ratio compared to controls, the difference was not statistically significant (p= 0.1). This suggests a dose-dependent effect of IL-2 on Treg proliferation. Treatment of the allografts with combination of CCL22 and IL2 resulted in reduced expression of IL-6, with grafts treated with CCL22+100 ng IL-2 delivered via MNs showing more than ten times reduction in IL-6, indicating reduced inflammation at the allograft site (p= 0.009) (**FIG. 13E**).

To study the systemic effects of MN-mediated local delivery of IL-2, Treg populations were evaluated in splenocytes harvested from allograft recipients. The data showed comparable Treg numbers for all the groups and no significant Treg expansion in the spleens (**FIG. 13F**). Systemic administration of IL-2 has been shown to promote Treg proliferation in the spleen but fail to do so in the skin allografts which aggravated their outcomes. Here, minimal systemic effects following MN-based delivery of IL-2 were confirmed, suggesting the enhanced safety of the MN-based platform when compared to systemic routes. The MN-based platform showed the potential to modulate immune cell composition and reduce inflammatory state locally. The MNs also proved their potential to extract ISF, which could be used as a diagnostic tool to monitor the response to therapy. Upon retrieval, intact MNs can be digested under reducing conditions thanks to a chemical modification introduced in the matrix, thereby allowing an on-demand recovery of infiltrated ISF in less than 5 minutes. Analysis of the entrapped ISF revealed a correlation between the Treg population present in ISF with that from the allograft (data not shown), boosting the potential of this platform and supplementing the therapeutic compartment.

### OTHER EMBODIMENTS

It is to be understood that while certain embodiments have been described within the detailed description, the present disclosure is intended to illustrate and not limit the scope of any embodiment defined by the scope of the appended claims.

## Claims

1. A microneedle array comprising:
a plurality of microneedles projecting from a substrate, each microneedle of the plurality of microneedles comprising a penetrating tip and a base that is integrally connected with the substrate,
wherein each microneedle of the plurality of microneedles is a porous microneedle composed of a degradable hyaluronic acid polymer comprising a disulfide bond coupled to a terminal amine group, and
wherein the hyaluronic acid polymer is crosslinked via the terminal amine group.

2. The microneedle array of claim 1, wherein each microneedle of the plurality of microneedles has a height of 100 µm (±10%) to 1,500 µm (±10%) and a base having a radius of 100 (±10%) µm to 1,500 µm (±10%); optionally, wherein each microneedle has a height of 600 µm (±10%) and a base having a radius of 150 µm (±10%).

3. The microneedle array of any one of claims 1 or 2, wherein the substrate is a polymeric, biodegradable substrate; optionally,
wherein the polymeric, biodegradable substrate comprises poly(D,L-lactide-co-glycolide) polymer.

4. The microneedle array of any one of claims 1-3, wherein each microneedle of the plurality of microneedles further comprises a therapeutic agent; optionally,
wherein the therapeutic agent comprises a chemokine, a chemotherapeutic, a nucleic acid, a protein, a macromolecule, a nanoparticle, a chemical-based drug, or any combination thereof.

5. A method of preparing a microneedle array, the method comprising:
providing a hyaluronic acid polymer solution comprising a disulfide bond coupled to a terminal amine group;
casting the hyaluronic acid polymer solution into a microneedle mold;
optionally centrifuging the microneedle mold containing the hyaluronic acid polymer solution; freeze-drying the microneedle mold containing the hyaluronic acid polymer solution;
casting a crosslinker into the microneedle mold containing the freeze-dried hyaluronic acid polymer;
centrifuging the microneedle mold containing the freeze-dried hyaluronic acid polymer and the crosslinker, thereby crosslinking the hyaluronic acid polymer and forming a hydrogel, wherein the hyaluronic acid polymer is crosslinked via the terminal amine group; and
optionally freeze-drying the microneedle mold containing the hyaluronic acid hydrogel.

6. The method of claim 5, further comprising depositing a solution comprising one or more therapeutic agents after optionally freeze-drying the microneedle mold containing the hyaluronic acid hydrogel; optionally,
wherein the solution comprising the one or more therapeutic agents further comprises glycine.

7. The method of any one of claims 5 or 6, wherein the crosslinker is polyethylene glycol (PEG) comprising a succinimidyl functional group; optionally,
wherein the PEG has a molecular weight ranging from 10 kDa (±10%) to 40 kDa (±10%); or,
wherein the chemical crosslinker comprises a first PEG having a molecular weight of 40 kDa (±10%) and a second PEG having a molecular weight of 10 kDa (±10%); optionally,
wherein the chemical crosslinker comprises the first PEG and the second PEG at a ratio of 0:100 wt% to 100:0 wt%.

8. A microneedle array of any one of claims 1-4 for use in a method of transdermally delivering a therapeutic agent to a subject in need thereof, the method comprising:
contacting the microneedle array of any one of claims 1-4 with a skin surface of the subject; and
applying pressure on the microneedle array such that the penetrating tip of each microneedle of the plurality of microneedles penetrates the skin surface, thereby releasing the therapeutic agent.

9. The microneedle array for use according to claim 8, further comprising maintaining the microneedle array in place for 5 minutes (±10%) to 24 hours (±10%) after the penetrating tip of each microneedle of the plurality of microneedles penetrates the skin surface.

10. The microneedle array for use according to claim 8 or 9, further comprising sampling a skin interstitial fluid of the subject, wherein the sampling step comprises extracting the skin interstitial fluid with the plurality of microneedles; optionally,
wherein the interstitial fluid comprises a biomarker and/or a cell; and/or,
wherein the therapeutic agent comprises a cell, a chemokine, a chemotherapeutic, a nucleic acid, a protein, a macromolecule, a nanoparticle, an exosome, a chemical-based drug, or any combination thereof.

11. A microneedle array of any one of claims 1-4 for use in a method of sampling an interstitial fluid of a subject in need thereof, the method comprising:
contacting the microneedle array of any one of claims 1-4 with a skin surface of the subject; and
applying pressure on the microneedle array such that the penetrating tip of each microneedle of the plurality of microneedles penetrates the skin surface, thereby absorbing the interstitial fluid.

12. The microneedle array for use according to claim 11, further comprising removing the microneedle array from the skin surface after the interstitial fluid is absorbed; and degrading the plurality of microneedles by contacting the plurality of microneedles with a reducing agent, thereby extracting the interstitial fluid from the plurality of microneedles; optionally,
wherein the disulfide bond is configured to be cleaved upon exposure to the reducing agent; and/or,
wherein the reducing agent is tris(2-carboxyethyl)phosphine (TCEP); optionally, wherein the disulfide bond is configured to be cleaved by exposure to 1 mM (±10%) to 100 mM (±10%) of TCEP; and/or,
wherein the reducing agent is glutathione, dithiothreitol, or beta-mercaptoethanol; and/or,
wherein the interstitial fluid comprises a biomarker and/or a cell; optionally,
wherein the biomarker comprises a cytokine, a chemokine, glucose, bilirubin, carnosine, cortisol, creatine, creatinine, homocysteine, uric acid, vitamin A, vitamin B12, lumazine, pantothenic acid (vitamin B5), lumichrome, pyridoxamine (vitamin B6 form), pyridoxal (vitamin B6 form), 4-pyridoxic acid, ascorbic acid (vitamin C), ergocalciferol (vitamin D2), 7-dehydrocholesterol (vitamin D3), hypoxanthine, nicotinamide adenine dinucleotide (NAD), uridine, xanthine, myristic acid (c14:0), palmitoleic acid (c16:1), stearic acid (c18:0), arachidic acid (c20:0), cholic acid, glycocholic acid, urocanic acid, 4-Guanidinobutanoic acid, succinylhomoserine, tocopherol, N6-(delta2-isopentenyl)-adenine, nebularine, cytidine monophosphate (CMP), cytidine, inosine, 3-methyladenine, nicotinamide ribotide, N-methyltryptamine, sphingosine, 20-COOH-leukotriene B4, stachyose, gulonolactone, fructose 6-phosphate, rhamnose, oxalic acid, phosphoenolpyruvic acid, diethanolamine, cyclohexane-1,2-diol, triethanolamine, methyl jasmonate, or any combinations thereof.

13. A hydrogel composition comprising:
a degradable hyaluronic acid polymer comprising the following chemical structure: 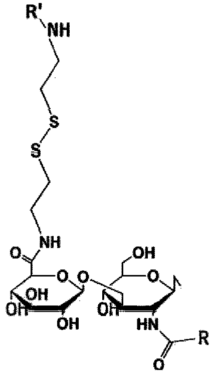
wherein the R group is a methyl group and the R' group is polyethylene glycol (PEG) crosslinker comprising a succinimidyl functional group.

14. The hydrogel composition of claim 13 for use in a method of delivering a therapeutic agent to a subject in need thereof, the method comprising:
contacting the subject with the hydrogel composition of claim 13, the hydrogel configured to be degraded upon exposure to a reducing agent,
wherein the hydrogel composition is crosslinked with a crosslinker comprising PEG further comprising a succinimidyl functional group; optionally,
wherein the hydrogel is a microneedle array, an injectable hydrogel, a pre-formed hydrogel depot, a sprayable hydrogel, or any combination thereof.

15. The microneedle array of any one of claims 1-4, wherein the degradable hyaluronic acid polymer comprises the following chemical structure: 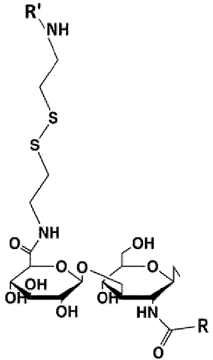 wherein the R group is a methyl group and the R' group is hydrogen.

## Patentansprüche

1. Mikronadelanordnung, umfassend:
eine Vielzahl von Mikronadeln, die von einem Substrat vorstehen, jede Mikronadel der Vielzahl von Mikronadeln umfassend eine penetrierende Spitze und eine Basis, die mit dem Substrat integral verbunden ist,
wobei jede Mikronadel der Vielzahl von Mikronadeln eine poröse Mikronadel ist, die aus einem abbaubaren Hyaluronsäurepolymer zusammengesetzt ist, umfassend eine Disulfidbindung, die mit einer terminalen Amingruppe gekoppelt ist, und
wobei das Hyaluronsäurepolymer über die terminale Amingruppe vernetzt ist.

2. Mikronadelanordnung nach Anspruch 1, wobei jede Mikronadel der Vielzahl von Mikronadeln eine Höhe von 100 µm (±10 %) bis 1.500 µm (±10 %) aufweist und eine Basis einen Radius von 100 (±10 %) µm bis 1.500 µm (±10 %) aufweist; optional
wobei jede Mikronadel eine Höhe von 600 µm (±10 %) aufweist und eine Basis einen Radius von 150 µm (±10 %) aufweist.

3. Mikronadelanordnung nach einem der Ansprüche 1 oder 2, wobei das Substrat ein polymeres, biologisch abbaubares Substrat ist; optional
wobei das polymere, biologisch abbaubare Substrat Poly(D,L-lactid-co-glycolid)-Polymer umfasst.

4. Mikronadelanordnung nach einem der Ansprüche 1 bis 3, wobei jede Mikronadel der Vielzahl von Mikronadeln ferner ein Therapeutikum umfasst; optional
wobei das Therapeutikum ein Chemokin, ein Chemotherapeutikum, eine Nukleinsäure, ein Protein, ein Makromolekül, ein Nanopartikel, ein chemisch-basiertes Arzneimittel oder eine beliebige Kombination davon umfasst.

5. Verfahren zum Herstellen einer Mikronadelanordnung, das Verfahren umfassend:
Bereitstellen einer Hyaluronsäurepolymerlösung, umfassend eine Disulfidbindung, die mit einer terminalen Amingruppe gekoppelt ist;
Gießen der Hyaluronsäurepolymerlösung in eine Mikronadelform;
optional Zentrifugieren der Mikronadelform, die die Hyaluronsäurepolymerlösung enthält;
Gefriertrocknen der Mikronadelform, die die Hyaluronsäurepolymerlösung enthält;
Gießen eines Vernetzers in die Mikronadelform, die das gefriergetrocknete Hyaluronsäurepolymer enthält;
Zentrifugieren der Mikronadelform, die das gefriergetrocknete Hyaluronsäurepolymer und den Vernetzer enthält, wodurch das Hyaluronsäurepolymer vernetzt und ein Hydrogel ausgebildet wird, wobei das Hyaluronsäurepolymer über die terminale Amingruppe vernetzt wird; und
optional Gefriertrocknen der Mikronadelform, die das Hyaluronsäurehydrogel enthält.

6. Verfahren nach Anspruch 5, ferner umfassend ein Abscheiden einer Lösung, umfassend ein oder mehrere Therapeutika, nach optionalem Gefriertrocknen der Mikronadelform, die das Hyaluronsäurehydrogel enthält; optional
wobei die Lösung, umfassend den einen oder die mehreren Therapeutika, ferner Glycin umfasst.

7. Verfahren nach einem der Ansprüche 5 oder 6, wobei der Vernetzer Polyethylenglycol (PEG) ist, umfassend eine Succinimidylfunktionsgruppe; optional
wobei das PEG ein Molekulargewicht in einem Bereich von 10 kDa (±10 %) bis 40 kDa (±10 %) aufweist; oder
wobei der chemische Vernetzer ein erstes PEG, das ein Molekulargewicht von 40 kDa (±10 %) aufweist, und ein zweites PEG umfasst, das ein Molekulargewicht von 10 kDa (±10 %) aufweist; optional
wobei der chemische Vernetzer das erste PEG und das zweite PEG in einem Verhältnis von 0 : 100 Gew.-% bis 100 : 0 Gew.-% umfasst.

8. Mikronadelanordnung nach einem der Ansprüche 1 bis 4 zur Verwendung in einem Verfahren zum transdermalen Abgeben eines Therapeutikums an ein Subjekt, das dies benötigt, das Verfahren umfassend:
Kontaktieren der Mikronadelanordnung nach einem der Ansprüche 1 bis 4 mit einer Hautoberfläche des Subjekts; und
Ausüben von Druck auf die Mikronadelanordnung derart, dass die penetrierende Spitze jeder Mikronadel der Vielzahl von Mikronadeln die Hautoberfläche penetriert, wodurch das Therapeutikum freigesetzt wird.

9. Mikronadelanordnung zur Verwendung nach Anspruch 8, ferner umfassend ein Halten der Mikronadelanordnung an Ort und Stelle für 5 Minuten (±10 %) bis 24 Stunden (±10 %), nachdem die penetrierende Spitze jeder Mikronadel der Vielzahl von Mikronadeln die Hautoberfläche penetriert.

10. Mikronadelanordnung zur Verwendung nach Anspruch 8 oder 9, ferner umfassend ein Probenehmen eines interstitiellen Hautfluids des Subjekts, wobei der Probeentnahmeschritt ein Extrahieren des interstitiellen Hautfluids mit der Vielzahl von Mikronadeln umfasst; optional
wobei das interstitielle Fluid einen Biomarker und/oder eine Zelle umfasst; und/oder
wobei das Therapeutikum eine Zelle, ein Chemokin, ein Chemotherapeutikum, eine Nukleinsäure, ein Protein, ein Makromolekül, ein Nanopartikel, ein Exosom, ein chemisch-basiertes Arzneimittel oder jeder Kombination davon umfasst.

11. Mikronadelanordnung nach einem der Ansprüche 1 bis 4 zur Verwendung in einem Verfahren zum Probenehmem eines interstitiellen Fluids eines Subjekts, das dies benötigt, das Verfahren umfassend:
Kontaktieren der Mikronadelanordnung nach einem der Ansprüche 1 bis 4 mit einer Hautoberfläche des Subjekts; und
Ausüben von Druck auf die Mikronadelanordnung derart, dass die penetrierende Spitze jeder Mikronadel der Vielzahl von Mikronadeln die Hautoberfläche penetriert, wodurch das interstitielle Fluid absorbiert wird.

12. Mikronadelanordnung zur Verwendung nach Anspruch 11, ferner umfassend ein Entfernen der Mikronadelanordnung von der Hautoberfläche, nachdem das interstitielle Fluid absorbiert ist; und Abbauen der Vielzahl von Mikronadeln durch Kontaktieren der Vielzahl von Mikronadeln mit einem Reduktionsmittel, wodurch das interstitielle Fluid aus der Vielzahl von Mikronadeln extrahiert wird; optional
wobei die Disulfidbindung konfiguriert ist, um bei Exposition gegenüber dem Reduktionsmittel gespalten zu werden; und/oder
wobei das Reduktionsmittel Tris(2-carboxyethyl)phosphin (TCEP) ist; optional
wobei die Disulfidbindung konfiguriert ist, um durch Exposition gegenüber 1 mM (±10 %) bis 100 mM (±10 %) TCEP gespalten zu werden; und/oder
wobei das Reduktionsmittel Glutathion, Dithiothreitol oder Beta-Mercaptoethanol ist; und/oder, wobei das interstitielle Fluid einen Biomarker und/oder eine Zelle umfasst; optional
wobei der Biomarker ein Cytokin, ein Chemokin, Glucose, Bilirubin, Carnosin, Cortisol, Creatin, Creatinin, Homocystein, Harnsäure, Vitamin A, Vitamin B12, Lumazin, Pantothensäure (Vitamin B5), Lumichrom, Pyridoxamin (Vitamin B6-Form), Pyridoxal (Vitamin B6-Form), 4-Pyridoxinsäure, Ascorbinsäure (Vitamin C), Ergocalciferol (Vitamin D2), 7-Dehydrocholesterol (Vitamin D3), Hypoxanthin, Nicotinamidadenindinucleotid (NAD), Uridin, Xanthin, Myristinsäure (c14:0), Palmitoleinsäure (c16:1), Stearinsäure (c18:0), Arachinsäure (c20:0), Cholsäure, Glycocholsäure, Urocansäure, 4-Guanidinobutansäure, Succinylhomoserin, Tocopherol, N6-(delta2-Isopentenyl)-adenin, Nebularin, Cytidinmonophosphat (CMP), Cytidin, Inosin, 3-Methyladenin, Nicotinamidribotid, N-Methyltryptamin, Sphingosin, 20-COOH-Leukotrien B4, Stachyose, Gulonolacton, Fructose-6-phosphat, Rhamnose, Oxalsäure, Phosphoenolpyruvsäure, Diethanolamin, Cyclohexan-1,2-diol, Triethanolamin, Methyljasmonat oder beliebige Kombinationen davon umfasst.

13. Hydrogelzusammensetzung, umfassend:
ein abbaubares Hyaluronsäurepolymer, umfassend die folgende chemische Struktur: 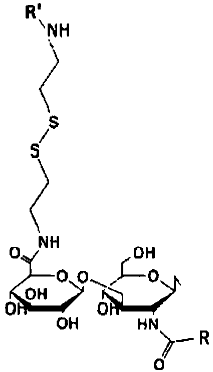
wobei die R-Gruppe eine Methylgruppe ist und die R'-Gruppe ein Polyethylenglycolvernetzer (PEG-Vernetzer) ist, umfassend eine Succinimidylfunktionsgruppe.

14. Hydrogelzusammensetzung nach Anspruch 13 zur Verwendung in einem Verfahren zum Abgeben eines Therapeutikums an ein Subjekt, das dies benötigt, das Verfahren umfassend:
Kontaktieren des Subjekts mit der Hydrogelzusammensetzung von Anspruch 13, wobei das Hydrogel konfiguriert ist, um bei Exposition gegenüber einem Reduktionsmittel abgebaut zu werden,
wobei die Hydrogelzusammensetzung mit einem Vernetzer vernetzt ist, umfassend PEG, ferner umfassend eine Succinimidylfunktionsgruppe; optional
wobei das Hydrogel eine Mikronadelanordnung, ein injizierbares Hydrogel, ein vorgeformtes Hydrogeldepot, ein sprühbares Hydrogel oder eine beliebige Kombination davon ist.

15. Mikronadelanordnung nach einem der Ansprüche 1 bis 4, wobei das abbaubare Hyaluronsäurepolymer die folgende chemische Struktur umfasst: 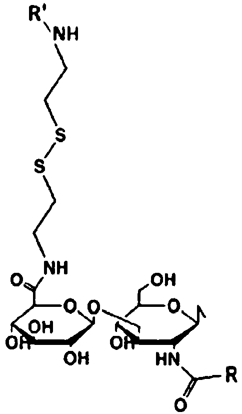 wobei die R-Gruppe eine Methylgruppe ist und die R'-Gruppe Wasserstoff ist.

## Revendications

1. Réseau de micro-aiguilles comprenant :
une pluralité de micro-aiguilles faisant saillie à partir d'un substrat, chaque micro-aiguille de la pluralité de micro-aiguilles comprenant une pointe pénétrante et une base qui est intégralement liée au substrat,
dans lequel chaque micro-aiguille de la pluralité de micro-aiguilles est une micro-aiguille poreuse composée d'un polymère d'acide hyaluronique dégradable comprenant une liaison disulfure couplée à un groupe amine terminal, et
dans lequel le polymère d'acide hyaluronique est réticulé par le groupe amine terminal.

2. Réseau de micro-aiguilles selon la revendication 1, dans lequel chaque micro-aiguille de la pluralité de micro-aiguilles a une hauteur de 100 µm (± 10 %) à 1 500 µm (± 10 %) et une base ayant un rayon de 100 (± 10 %) µm à 1 500 µm (± 10 %) ; facultativement,
dans lequel chaque micro-aiguille a une hauteur de 600 µm (± 10 %) et une base ayant un rayon de 150 µm (± 10 %).

3. Réseau de micro-aiguilles selon l'une quelconque des revendications 1 ou 2, dans lequel le substrat est un substrat polymère biodégradable ; facultativement,
dans lequel le substrat polymère biodégradable comprend un polymère de poly(D,L-lactide-co-glycolide).

4. Réseau de micro-aiguilles selon l'une quelconque des revendications 1 à 3, dans lequel chaque micro-aiguille de la pluralité de micro-aiguilles comprend en outre un agent thérapeutique ; facultativement,
dans lequel l'agent thérapeutique comprend une chimiokine, un agent chimiothérapeutique, un acide nucléique, une protéine, une macromolécule, une nanoparticule, un médicament à base de produits chimiques, ou toute combinaison de ceux-ci.

5. Procédé de préparation d'un réseau de micro-aiguilles, le procédé comprenant :
la fourniture d'une solution de polymère d'acide hyaluronique comprenant une liaison disulfure couplée à un groupe amine terminal ;
le coulage de la solution de polymère d'acide hyaluronique dans un moule à micro-aiguilles ;
la centrifugation facultative du moule à micro-aiguilles contenant la solution de polymère d'acide hyaluronique ;
la lyophilisation du moule à micro-aiguilles contenant la solution de polymère d'acide hyaluronique ;
le coulage d'un agent de réticulation dans le moule à micro-aiguilles contenant le polymère d'acide hyaluronique lyophilisé ;
la centrifugation du moule à micro-aiguilles contenant le polymère d'acide hyaluronique lyophilisé et l'agent de réticulation, permettant ainsi de réticuler le polymère d'acide hyaluronique et de former un hydrogel, dans lequel le polymère d'acide hyaluronique est réticulé par le groupe amine terminal ; et
la lyophilisation facultative du moule à micro-aiguilles contenant l'hydrogel d'acide hyaluronique.

6. Procédé selon la revendication 5, comprenant en outre le dépôt d'une solution comprenant un ou plusieurs agents thérapeutiques après la lyophilisation facultative du moule à micro-aiguilles contenant l'hydrogel d'acide hyaluronique ; facultativement,
dans lequel la solution comprenant le ou les agents thérapeutiques comprend en outre de la glycine.

7. Procédé selon l'une quelconque des revendications 5 ou 6, dans lequel l'agent de réticulation est le polyéthylène glycol (PEG) comprenant un groupe fonctionnel succinimidyle ; facultativement,
dans lequel le PEG a une masse moléculaire allant de 10 kDa (± 10 %) à 40 kDa (± 10 %) ; ou,
dans lequel l'agent de réticulation chimique comprend un premier PEG ayant une masse moléculaire de 40 kDa (± 10 %) et un second PEG ayant une masse moléculaire de 10 kDa (± 10 %) ; facultativement,
dans lequel l'agent de réticulation chimique comprend le premier PEG et le second PEG à un rapport de 0:100 % en poids à 100:0 % en poids.

8. Réseau de micro-aiguilles selon l'une quelconque des revendications 1 à 4 pour une utilisation dans un procédé d'administration transdermique d'un agent thérapeutique à un sujet en ayant besoin, le procédé comprenant :
la mise en contact du réseau de micro-aiguilles selon l'une quelconque des revendications 1 à 4 avec une surface cutanée du sujet ; et
l'application de pression sur le réseau de micro-aiguilles de telle sorte que la pointe pénétrante de chaque micro-aiguille de la pluralité de micro-aiguilles pénètre dans la surface de la peau, permettant ainsi de libérer l'agent thérapeutique.

9. Réseau de micro-aiguilles pour une utilisation selon la revendication 8, comprenant en outre le maintien du réseau de micro-aiguilles en place pendant 5 minutes (± 10 %) à 24 heures (± 10 %) après que la pointe pénétrante de chaque micro-aiguille de la pluralité de micro-aiguilles a pénétré dans la surface de peau.

10. Réseau de micro-aiguilles pour une utilisation selon la revendication 8 ou 9, comprenant en outre l'échantillonnage d'un fluide interstitiel cutané du sujet, dans lequel l'étape d'échantillonnage comprend l'extraction du fluide interstitiel cutané avec la pluralité de micro-aiguilles ; facultativement,
dans lequel le fluide interstitiel comprend un biomarqueur et/ou une cellule ; et/ou,
dans lequel l'agent thérapeutique comprend une cellule, une chimiokine, un agent chimiothérapeutique, un acide nucléique, une protéine, une macromolécule, une nanoparticule, un exosome, un médicament à base de produits chimiques, ou toute combinaison de ceux-ci.

11. Réseau de micro-aiguilles selon l'une quelconque des revendications 1 à 4 pour une utilisation dans un procédé d'échantillonnage d'un fluide interstitiel d'un sujet en ayant besoin, le procédé comprenant :
la mise en contact du réseau de micro-aiguilles selon l'une quelconque des revendications 1 à 4 avec une surface cutanée du sujet ; et
l'application de pression sur le réseau de micro-aiguilles de telle sorte que la pointe pénétrante de chaque micro-aiguille de la pluralité de micro-aiguilles pénètre dans la surface de la peau, permettant ainsi d'absorber le fluide interstitiel.

12. Réseau de micro-aiguilles pour une utilisation selon la revendication 11, comprenant en outre le retrait du réseau de micro-aiguilles de la surface cutanée après que le fluide interstitiel a été absorbé ; et la dégradation de la pluralité de micro-aiguilles par mise en contact de la pluralité de micro-aiguilles avec un agent réducteur, permettant ainsi d'extraire le fluide interstitiel de la pluralité de micro-aiguilles ; facultativement,
dans lequel la liaison disulfure est conçue pour être clivée lors de l'exposition à l'agent réducteur ; et/ou,
dans lequel l'agent réducteur est la tris(2-carboxyéthyl)phosphine (TCEP) ; facultativement,
dans lequel la liaison disulfure est conçue pour être clivée par une exposition à 1 mM (± 10 %) à 100 mM (± 10 %) de TCEP ; et/ou,
dans lequel l'agent réducteur est le glutathion, le dithiothréitol, ou le bêta-mercaptoéthanol ; et/ou, dans lequel le fluide interstitiel comprend un biomarqueur et/ou une cellule ; facultativement,
dans lequel le biomarqueur comprend une cytokine, une chimiokine, du glucose, de la bilirubine, de la carnosine, du cortisol, de la créatine, de la créatinine, de l'homocystéine, de l'acide urique, de la vitamine A, de la vitamine B12, de la lumazine, de l'acide pantothénique (vitamine B5), du lumichrome, de la pyridoxamine (forme de vitamine B6), du pyridoxal (forme de vitamine B6), de l'acide 4-pyridoxique, de l'acide ascorbique (vitamine C), de l'ergocalciférol (vitamine D2), du 7-déshydrocholestérol (vitamine D3), de l'hypoxanthine, du nicotinamide adénine dinucléotide (NAD), de l'uridine, de la xanthine, de l'acide myristique (C14:0), de l'acide palmitoléique (C16:1), de l'acide stéarique (C18:0), de l'acide arachidique (C20:0), de l'acide cholique, de l'acide glycocholique, de l'acide urocanique, de l'acide 4-guanidinobutanoïque, de la succinylhomosérine, du tocophérol, de la N6-(delta2-isopentényl)-adénine, de la nébularine, du cytidine monophosphate (CMP), de la cytidine, de l'inosine, de la 3-méthyladénine, du nicotinamide ribotide, de la N-méthyltryptamine, de la sphingosine, du 20-COOH-leucotriène B4, du stachyose, de la gulonolactone, du fructose 6-phosphate, du rhamnose, de l'acide oxalique, de l'acide phosphoénolpyruvique, de la diéthanolamine, du cyclohexane-1,2-diol, de la triéthanolamine, du jasmonate de méthyle, ou toutes combinaisons de ceux-ci.

13. Composition d'hydrogel comprenant :
un polymère d'acide hyaluronique dégradable comprenant la structure chimique suivante : 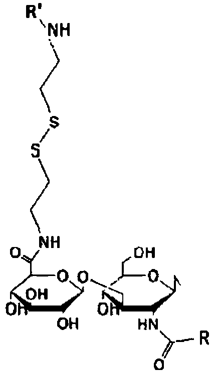
dans laquelle le groupe R est un groupe méthyle et le groupe R' est un agent de réticulation de polyéthylène glycol (PEG) comprenant un groupe à fonctionnalité succinimidyle.

14. Composition d'hydrogel selon la revendication 13 pour une utilisation dans un procédé d'administration d'un agent thérapeutique à un sujet en ayant besoin, le procédé comprenant :
la mise en contact du sujet avec la composition d'hydrogel selon la revendication 13, l'hydrogel étant conçu pour être dégradé lors de l'exposition à un agent réducteur,
dans laquelle la composition d'hydrogel est réticulée avec un agent de réticulation comprenant du PEG comprenant en outre un groupe à fonctionnalité succinimidyle ; facultativement,
dans laquelle l'hydrogel est un réseau de micro-aiguilles, un hydrogel injectable, un dépôt d'hydrogel préformé, un hydrogel pulvérisable, ou toute combinaison de ceux-ci.

15. Réseau de micro-aiguilles selon l'une quelconque des revendications 1 à 4, dans lequel le polymère d'acide hyaluronique dégradable comprend la structure chimique suivante : 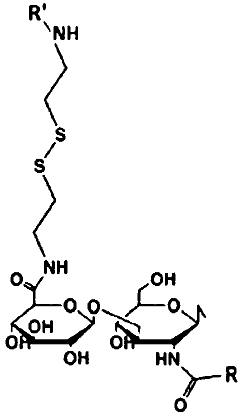 dans laquelle le groupe R est un groupe méthyle et le groupe R' est l'hydrogène.
